(19) Europäisches Patentamt
European Patent Office
Office européen des brevets

(11) EP 1 336 661 A1

(12) EUROPEAN PATENT APPLICATION

(43) Date of publication:
**20.08.2003 Bulletin 2003/34**

(51) Int Cl.[7]: **C12Q 1/68**

(21) Application number: **02003401.3**

(22) Date of filing: **14.02.2002**

(84) Designated Contracting States:
**AT BE CH CY DE DK ES FI FR GB GR IE IT LI LU MC NL PT SE TR**
Designated Extension States:
**AL LT LV MK RO SI**

(71) Applicant: **Biofrontera Pharmaceuticals AG**
**51377 Leverkusen (DE)**

(72) Inventors:
- **Lübbert, Hermann, Prof. Dr.**
  **51381 Leverkusen (DE)**
- **Engels, Peter, Dr.**
  **51429 Bergisch Gladbach (DE)**
- **Schmitz, Beate, Dr.**
  **50825 Köln (DE)**

(74) Representative:
**Sternagel, Fleischer, Godemeyer & Partner**
**Patentanwälte**
**Braunsberger Feld 29**
**51429 Bergisch Gladbach (DE)**

(54) **Multiple genes relevant for the characterisation, diagnosis, and manipulation of neuropatic pain**

(57) The present invention relates to the use of differentially expressed polynucleotide sequences or polypeptides for the characterisation of pain status or progression, a method for characterising pain, a method for identifying therapeutic agents for pain and the use of such sequences for the development of a medicament.



Printed by Jouve, 75001 PARIS (FR)

<110> Biofrontera Pharmaceuticals AG

<120> Multiple Genes relevant for the characterisation, diagnosis and manipulation of neuropathic pain

<130> P10852EPalleSeq

<140>
<141>

<160> 64

<170> PatentIn Ver. 2.1

<210> 1
<211> 193
<212> DNA
<213> Rattus norvegicus

<400> 1
acgcgagaga ctcagtacca actcctcaac taaccttata atgactgcat ggatgccccc 60
caccctatca cacattcgaa gaaccttcct acgtaaaagt taaataagaa aggaaggatt 120
cgaacccccct acaactggtt tcaagccaat ttcataacca ttatgtcttt ctcaataaaa 180
aaaaaaaaaa aaa 193

<210> 2
<211> 244
<212> DNA
<213> Rattus norvegicus

<400> 2
cttattgatt gggagtgacc atatgatgca agctgtacag agtgctggtg gcgtgcctgg 60
aggaatgctg gtggctgccc tgggtggagg agctcctcca tctggatggt gcttcttcag 120
gccccaccat tgaagaggtc gattaagtca aagtagaggg tatagcattg ttccacaggg 180
acccaaaaca agtaacatgg aataataaaa ctatttaaat tggcaccaaa aaaaaaaaaa 240
aaaa 244

<210> 3
<211> 155
<212> DNA
<213> Rattus norvegicus

<400> 3
tccatatcgc atgatcctcg cttaagttag ggattgagca cgacgaagca ttttagcgat 60
tcggatggtc gcacttgact atgtagtttc cagtgcacag agcgacctaa tggctcaata 120
aacttggcct caacagtcaa aaaaaaaaaa aaaaa 155

<210> 4
<211> 132
<212> DNA
<213> Rattus norvegicus

<400> 4
gcggttgttn tcggggaggg ctagcgacaa ctgactgatc tattaaacaa agcatccgat 60
cgtccgcggt cgggtgttga cgcgatgtga tttctagccc agtgctctag aatgtcaaag 120
aaaaaaaaaa aa 132

<210> 5
<211> 85
<212> DNA
<213> Rattus norvegicus

<400> 5
gtcnaccatt gtactagaaa ctagtgctct aaaatatttc gaaaactgat cagcttctat 60
aaatttaaac caaaaaaaaa aaaaa 85

<210> 6
<211> 100
<212> DNA
<213> Rattus norvegicus

<400> 6
agaacaggtg agntgtcggc agcacgcggc atacgattac ccacactaat tattttcggc 60
gtaaaacgtg ccaactataa atctcaaaaa aaaaaaaaaa 100

<210> 7
<211> 178
<212> DNA
<213> Rattus norvegicus

<400> 7
cctgattgtg atgagctgta ctgttcgtta gttagcgact attagtctta cttaatctgt 60
acttctgatc tggagcattc gaggtcggtt tctatctatt gtacaatttc tgccctagta 120
cgtacggacc cgagaaatgg agcctcctat accataattg ctcccaacca aaaaaaaa 178

<210> 8
<211> 85
<212> DNA
<213> Rattus norvegicus

<400> 8
tggcagcacg nggacatacg anntacccan attaattatt tactggcgta aacgtgccaa 60
ctatacatct caacaaaaaa aaaaa 85

<210> 9
<211> 167
<212> DNA
<213> Rattus norvegicus

<400> 9
ggctcatttt ctcacaccca cgtgacacac gtcatacgtt tgtgtatact gtgcaggcaa 60
atgctcactc tccccggaaa gagaattttg aatgtggaaa ggagaaaggc agaggaggaa 120
gaaaattctt ttaatttaaa gcacacattc aaaaaaaaaa aaaaaaa 167

<210> 10
<211> 150
<212> DNA
<213> Rattus norvegicus

<400> 10
caaatcagtc gtcctctact cctccatccc ttctccccct ccattccgct ctagctatgt 60
gaaacttcag tggttagaaa ttaaagacct cgcagttatg tgcaataaat agtaaataca 120
agttacagtg gatgacaaaa aaaaaaaaaa 150

<210> 11
<211> 255
<212> DNA
<213> Rattus norvegicus

<400> 11
gggaggagcg accgacacga actagaaatt atgagcacaa gtacactgga atcctccagc 60
ttcagagctg cttcctccac ccacagacct gcttcctcct ccaccgtgcc cgagcaggcc 120
taacctccag accgtcagag aggacagcta tggtctagga cagttctggt gttaccctgg 180

```
agtccacggg aggggaacta gtccagactg cctgagatga gtaaagtatc tggcgtcacc 240
aaaaaaaaaa aaaaa                                                  255

<210> 12
<211> 158
<212> DNA
<213> Rattus norvegicus

<400> 12
acacccanac cgtagacaat acctacctnc cacagcctac ttgtcctgtc tctagctact 60
actccacgca gatactgcgt gctcctctcc aaactgactc gtcctcatta acagtcaaca 120
ttaaactaac agcattaaca caaaaaaaaa aaaaaaaa                         158

<210> 13
<211> 116
<212> DNA
<213> Rattus norvegicus

<400> 13
caggcgctgt ctaagttact ttgtattgtt ttccgtacta ctaatactgt atgttgcctg 60
gtgccaacaa atacttcaaa atacatttgt tgtaaatgca aaaaaaaaaa aaaaaa     116

<210> 14
<211> 325
<212> DNA
<213> Rattus norvegicus

<400> 14
gcgccaagca tccgtgctag atagtgtctc gcctagagac ggccattcct gagaaaggga 60
gaccgggact gatcgttctc atcctcaggg gcagagttgg ccccaccacg ggccatgtgg 120
gttctaaatg agggtagtgt tccagtgacc tgagacgcca cagctgtgag ctccacgtcg 180
tgccggtacg cgactgacac cgacctgggt catgaccctg cttcgcagtt cctcctcaca 240
ttatcctcct ttgccgacac gcacctacta tcggtctcaa ctcttcttat aaatgattca 300
catacctgtg aaaaaaaaaa aaaaa                                       325

<210> 15
<211> 97
<212> DNA
<213> Rattus norvegicus

<400> 15
gtggtttatc tatttacaat ttctcccagt ttcgaaagga caagagaaat ggagcctcct 60
taccataagt gctcccaacc aaaaaaaaaa aaaaaaa                          97

<210> 16
<211> 76
<212> DNA
<213> Rattus norvegicus

<400> 16
ctgggcacna nccnanaatg taacttacct ataacctnaa agagggacag ctctttagga 60
aaaacaaaaa aaaaaa                                                 76

<210> 17
<211> 167
<212> DNA
<213> Rattus norvegicus

<400> 17
cgtntgatgc gngacnacga gggcggcagg ggcntnccna tcgtgccgct anatggtaat 60
gaccgtggct ggtacaacga cggaccagag cgacagcatt cgccaggaat gtttaacatt 120
acatcnaaaa aaancaaaan acgctcncca cnaacaccna aaaaaaa               167
```

<210> 18
<211> 181
<212> DNA
<213> Rattus norvegicus

<400> 18
atgaaattat agagatgcgt cgatctatag cgtgagttga tggactactg tctggcaggt 60
catgttacgg attgacgcat accacagtgt gagaaactcc aaggatatca gacacgtagg 120
agtcaagcac aaagcccgat attctaattt acttaagaat gtccaanaaa aaaaaaaaaa 180
a 181

<210> 19
<211> 153
<212> DNA
<213> Rattus norvegicus

<400> 19
gaaatgatgg gttgcacccc cagatngttt anagacccat ctgccataga ngtacngnaa 60
actantagta ccatntctnt caaataaaga ataancnggt aggcgaagcg atgnnnacaa 120
nacacacnaa cnaaaacaaa aanaaaacgc aaa 153

<210> 20
<211> 97
<212> DNA
<213> Rattus norvegicus

<400> 20
ttgtgctgca gtagcaaggc ctggaacagg acacagaatg ggctcgtgtg gaaggactgg 60
gacaataaag tgggtaaacc aaaaaaaaaa aaaaaaa 97

<210> 21
<211> 138
<212> DNA
<213> Rattus norvegicus

<400> 21
gcacctgagg agggacggcg cactctgacc accctccggg actgtatcac cacaagattc 60
ttatagcact agtatttttct gtattaaagt ttgcatggtt tctaataaag aattcaaacc 120
taaaaaaaaa aaaaaaaa 138

<210> 22
<211> 209
<212> DNA
<213> Rattus norvegicus

<400> 22
gctggggcgg attacctagt taattttctc tggtcaccca tacacagtac gtgggctcga 60
tcttttaatt ttgtatagtn gcactgtgtg cgtttcatac agtgtggctn gactgaaagt 120
tgtgaatgat ttgtcaggag acccgagacc gtccagttca ctgatgggtt tnaaataaaa 180
tactccctga tcttaaaaaa aaaaaaaaa 209

<210> 23
<211> 359
<212> DNA
<213> Rattus norvegicus

<400> 23
cctggtgtga ctgaggcatg ggcagtggac ggatgcccat gccgtgcttt gcgccaggct 60
actgtgaatg gtggaagcag gagggagagc cacctggtac tttcgtccct gcctcctcct 120
ccgttccgga tctctcccct ctgtccaggg gtgtgtgacg tttctcctgc atgtttttac 180

tgatgttcgt gctggccgcc ctcagcccgg agcctgggag aggctttggt gcctcgcatc 240
agacttcggt gctccgtggc ggtgaagccc ttaaatgctt tgtatatttt ctctattaga 300
tctcttttca gaagtgtctg tagaagatta aaaaaaacaa aaacaaaaaa aaaaaaaaa 359

<210> 24
<211> 167
<212> DNA
<213> Rattus norvegicus

<400> 24
cgtgggagcg tgaggagcgt agactgtggc gtgaagctcc gatcccaatt cgcggtggtg 60
gggaccaaca gactagcttc atcctgtcct tgtagctgta gctgtacccc tgaagctcag 120
ccagctctga ctttataaaa cataaaacct caaaaaaaaa aaaaaaa 167

<210> 25
<211> 121
<212> DNA
<213> Rattus norvegicus

<400> 25
aagacagaac gacaacnagg accgtagaag cgtgtgggtt gggcggagcc tacgggttct 60
acgcatgcta caccatatgt gcttagtaaa tgtctgttca atcgaaaaaa aaaaaaaaaa 120
a 121

<210> 26
<211> 245
<212> DNA
<213> Rattus norvegicus

<400> 26
gttggcagct gcaggatctg aaattgcatg ctgatggatg atctggaccg taacaaggat 60
caggaagtaa acttccagga gtatgttgcc ttcctggggg ccttggcttt gatctacaat 120
gaagctctga aataaaatgg gaaggcagag atgccttctg ggaggctatc ccagtcaagt 180
ccagtggggg ggtaattata caataaatat ttcgtttttg ttatgtctaa aaaaaaaaaa 240
aaaaa 245

<210> 27
<211> 113
<212> DNA
<213> Rattus norvegicus

<400> 27
agacgncgac gttcagtnac tacgatggga cggattcgtc atgcccctac aatctggttt 60
caagcccctt tcactaacca ttatgtcttt cgcaancaca aaaaaaaaaa aaa 113

<210> 28
<211> 254
<212> DNA
<213> Rattus norvegicus

<400> 28
gtccanccna cnatatgact tgtgccgctc gcatctgctn tcgtacccca gcttccngct 60
tttctcccac atactagaac tgtccagtcc tatgtagatg tagtctgacc taggactcta 120
tcctgaagga gctccctagg caggaatatg gtcccctatt cagacactag gccaggtgtg 180
actggggctc tctnagtggc cctcttagtg natgtgttgg caaccttaat aaatctagtg 240
gcagtggcaa aaaa 254

<210> 29
<211> 135
<212> DNA
<213> Rattus norvegicus

<400> 29
tgacagaggc agcctacctc gtgtctgtac tgctctctat ggtctctttg gttctgtaag 60
tgacggcctg gatgtggttg tctagtcctc agaggaagaa taaaactttg ctgctggcaa 120
aaaaaaaaaa aaaaa 135

<210> 30
<211> 462
<212> DNA
<213> Rattus norvegicus

<400> 30
aggtgacggc accaactgct tatacggact gcaggcgtct gatccagcct tccattgtac 60
ctgaactttg gctctaaggt tgggaatgtg gaatagtggt gccggacatt tctgccatga 120
taaccagtcc tgttgtaaag acagcgcaag ggaggctggg cctgctgtcc ctatgtaaag 180
ggtgtctgct gtagagatgg acgtcactgt agtcccattg gcttccactg ttcagccaag 240
ggaaccaagt gtttgcggaa gaagacccct cgctgggaca tacttttgag ggatccagcc 300
ccaagaccgc tactgtgagg aagggctaac gactaaagaa ctccacagtc ctgggaaccc 360
tgttctgagg gtatccacca ctcaggcctc cctggcacct cttcctttag tctccccggc 420
ctactcattc tgagtcaccc catcaccatg gaaggtgggg cc 462

<210> 31
<211> 208
<212> DNA
<213> Rattus norvegicus

<400> 31
gggtaggcgc ttcagaaagg tggtggctgg agtggcagtg ccctggctca caagtaccac 60
taaacctctt ttcctgctct tgtcttgtgc aatggtcaat tgttcccaag agagcatctg 120
tcagttgttg tcaaaatgac aaagaccttt gaaaatctgt cctactaata aaaggcattt 180
actttcactg caaaaaaaaa aaaaaaaa 208

<210> 32
<211> 211
<212> DNA
<213> Rattus norvegicus

<400> 32
gggtagngng aagaaacgng antnaccgtc ttanaactcg catttacgct caacacnctt 60
ntncccgacg cgagctttct natgcaagag gttatgnttc cggccggatc atctaggcca 120
aatggtgtgc aagactgagn aagggaagga agggagaaaa gcgcgtactc ctactttaga 180
gagtagctgg ttgcccngca aaaaaaaaaa a 211

<210> 33
<211> 190
<212> DNA
<213> Rattus norvegicus

<400> 33
cagccagcgg ctaggcgccg acaccccctg acggcttcgc agataagtta ctatttaaca 60
acgatttaac aggcaactag aactctaaac ttaatagagt agcttactag agataataat 120
attttactat taagatttta aaagacacag tatggaggtt tatttaaaca atttgaaaaa 180
aaaaaaaaaa 190

<210> 34
<211> 82
<212> DNA
<213> Rattus norvegicus

<400> 34
agagagacna ggtagtctcc cttactagcc ttcancttat ataaaaacaa cctaatgggc 60
taaaacaata aaaaaaaaaa aa 82

<210> 35
<211> 167
<212> DNA
<213> Rattus norvegicus

<400> 35
agactttgct ctgtgtcagc atgtaagtac tcactcctga tgcagtcctc catcgctgtt 60
gggaaagagg aatctatggc atagagcctg tgcttatcac taacattttg aaatttccaa 120
accctttagt taaataaatc tcttcttcct aaaaaaaaaa aaaaaaa 167

<210> 36
<211> 247
<212> DNA
<213> Rattus norvegicus

<400> 36
gagagagcna ctccanattc tncaccttct ttnttnntat ttagggccca ncctgcagtc 60
ncttctctct gnattctgca aagctgcagg ggctgaggat tcgagcacgt ctcggnggag 120
gnggctggct ccgtagcggc actgatnact gatgtatcta ttgagcacgg tccaatnatt 180
atctttangt atgcagctgg gaataaagta gaccccatgt gttagggggt ccgaaaacaa 240
aaaaaac 247

<210> 37
<211> 233
<212> DNA
<213> Rattus norvegicus

<400> 37
tgtgagcctc cgacgcttta gctcaccccc tcactctaca ctgagaataa gaatctgagt 60
gtgaacttga ttgttacaca taccttgaca caagtgtgga tagcttttca aattactgga 120
tggaatactt agagcgtttg ttgttgttgg ggtattagct attgttcttg tatttgtcct 180
ttgaaaacaa ataaactggc acgatgaacg aagccttcca acaaaaacaa aaa 233

<210> 38
<211> 118
<212> DNA
<213> Rattus norvegicus

<400> 38
gggcgacgga catcaggcgg tcctatctat ttacatttct cccagtacga aaggacaaga 60
gaaatggagc ctccttacca taagtgctcc caaccaattt atgaaaaaaa aaaaaaaa 118

<210> 39
<211> 118
<212> DNA
<213> Rattus norvegicus

<400> 39
agagagacta cggagcatca ggtcggttct atctatttac aatttctccc agtacgaaag 60
gacaagagaa atggagcctc cttaccataa gtgctcccaa ccaaaaaaaa aaaaaaaa 118

<210> 40
<211> 299
<212> DNA
<213> Rattus norvegicus

<400> 40
aacgcgtgca gcggagtcac ggagaccggc tgatggcatg gagatcgtaa cgcttatgct 60
gtgttatatg acatcatcct gaagaacttt gagaagctca agaagccccg tggagagaca 120
agaggggatg atctattgag ccccctctct cgtactatga tgggtataac agaaaccttc 180
ctactcttga ctaggaactc tagactgcac ccagtcttct tcctgctggg gtctcctccc 240
tcactctgcc ttccaaacac aataaataca tagttgccca ccaaaaaaaa aaaaaaaaa 299

<210> 41
<211> 112
<212> DNA
<213> Rattus norvegicus

<400> 41
aagttttagt ttaaccaata tttagtcagc tattcagttg caatcaatct ntatacgaga 60
ttcattctaa caaatntctc tcatgtacca cacacacana aaaaaaaaaa aa 112

<210> 42
<211> 336
<212> DNA
<213> Rattus norvegicus

<400> 42
gtgcggcaag tgagacacgt gataactgct gtgtgcctgt ccactctgag cgacctgcat 60
gcccacatac tgcgtgtgga tcctgtcaac ttcaagttcc tgagccactg cctgctggtg 120
accttggctt gccaccaccc tggagatttc acacccgcca tgcacgcctc tctggacaaa 180
ttccttgcct ctgtgagcac tgtgctgacc tccaagtacc gttaagccgc ctcctgccgg 240
gcttgccttc tgaccaggcc cttcttccct cccttgcacc tatacctctt ggtctttgaa 300
taaagcctga gtaggaagca aaaaaaaaaa aaaaaa 336

<210> 43
<211> 295
<212> DNA
<213> Rattus norvegicus

<400> 43
ggtcgtattc tatatcagat gcttacacca catgaaatac agtctcctct ataggctcat 60
tcatctcact tacggccgtc cttgtaatga tcttcatgat ttgagaagcc ttcgcatcaa 120
aacgagaagt actctcaatt tcctactcct caatataacc ctagaatgac tgcatggatg 180
ccccccaccc taccacacat tcgaagaacc ttcctacgta aaagttaaat aagaaaggaa 240
ggattcgaac cccctacaac tggggttntc cagagggcgc gaaattntct cacta 295

<210> 44
<211> 129
<212> DNA
<213> Rattus norvegicus

<400> 44
agtcgctgga gtaccaacta gtatgcggcg cttacgtgct actcatcctc tcctactata 60
acattcacgc atccaactta tatgtgaaaa ttcatggtta ggacctaagc ccaaaaaaaa 120
aaaaaaaaa 129

<210> 45
<211> 230
<212> DNA
<213> Rattus norvegicus

<400> 45
aagccgggac accaagctaa gtgctatccg cttgctagct gtagcttgag catcctcctg 60
tatctctgat aatgtgcgat ctccgagagg aacagaactg tcatataagc gaagttgagc 120
cttactgatc ccgtgggcga agttgcgacg ggacgctgag caactagacc ggtcggcagg 180
agtgagactt aggtgccttc tagatagttg tgacttaaaa aaaaaaaaaa 230

<210> 46
<211> 119
<212> DNA
<213> Rattus norvegicus

<400> 46
cctaacanaa natagaggca gaggcnggag gattctgagt tgaaggcagc ctggtctaca 60
aagtgagttt caggactgcc aggggctatc tcaaaacaaa aaaccaaaaa acaaaacaa 119

<210> 47
<211> 122
<212> DNA
<213> Rattus norvegicus

<400> 47
cttaancagt gtataagagg cagaggcngg aggtntctga gttgaaggcc agcctggtct 60
acaaagtgag tttcaggact gccagggcta tctcaaaaca aaaaaccaaa aaaaaaaaaa 120
aa 122

<210> 48
<211> 301
<212> DNA
<213> Rattus norvegicus

<400> 48
atcgtcgaca taatcttacc ccctatcttt tcagcttgtc atagacatca tcctcatccg 60
tcgtcaagag cttcaacagg aatgagtgtt agacccaaag gtcctgaggt gccacctgct 120
ccgccagctc cttccaatct tccctcctaa ggtcttggag acttccccac aagcgaccta 180
ccactgttgc ggtgctccaa acctcctccc cacctcatcc tccttccttt ccttggcttt 240
gatcatgcta atatttgggg aatattaaat aaagtgaatc tttgcacttg aaaaaaaaaa 300
a 301

<210> 49
<211> 473
<212> DNA
<213> Rattus norvegicus

<400> 49
accaagattt atggcgatac ttgtgacaat gcgagttggt tagttgtagg caactagtta 60
cacttggctc tgaatccttg gactcacggc aatgacctgt aactctacaa gagacactga 120
aacagtgaga gagggacttc cataccactg ggcaggctac aggcgcgtct cagttgtgac 180
ggtctattcc tggttgctca gtccccaacc tcgcgtcacc ctgggaccgc cgattctcaa 240
cctgaaagag tccacaacca tccttctgag tgccctccat ccccacaaac cactagcatg 300
ttgtactcca agcccaaggg gccccattcc ctttcttatg ccatgtcacg gagtatcagg 360
ctcagcactt caagcgtcca tcctggtttg aacagtttgg gcaaactgac accacgtagt 420
gtaacaccca tgcctggttg tgcagtgcca tccgtcattc gtgtaccctc act 473

<210> 50
<211> 216
<212> DNA
<213> Rattus norvegicus

<400> 50
aggaatacat gcngtactcn attttttgtt nngtttcttt atctgatggn catgattaac 60
gagtggacgg ccgggagnat nccgtattag cgccgttagg caggtagaac attcttgtgg 120
acccggcgca atagacggac caagactcga acaggccctt acgccaacgg aaactggttg 180
tccaccttaa ggtcaacaaa aacacacaca cacccc 216

<210> 51
<211> 446
<212> DNA
<213> Rattus norvegicus

<400> 51
gctgtctgtg acattccacc tcggggccta aaaatgtccg ccaccttcat tggcaacagc 60
acggctattc aggagctgtt caaacgcatc tcagagcagt tcacagccat gttccgacgc 120
aaggccttcc tacactggta cacgggcgag ggcatggatg agatggagtt cactgaggct 180

gagagcaaca tgaatgacct ggtgtccgag taccagcagt accaggatgc tacagctgag 240
gaggaaggag agtttgagga ggaggctgag gaggaggtgg cttagagctg tcttagtcac 300
tacagcatgg gagcagtgtg aactctttat tcattcacag cttgtctgct agccatgtcc 360
actgtgcatt tgctgtcctg tgtcctgaca tcacttgtac agataccacc attaaagcaa 420
ttcatagtgt caaaaaaaaa aaaaaa 446

<210> 52
<211> 276
<212> DNA
<213> Rattus norvegicus

<400> 52
gcgcagtacc tagggataca gcgcatccta tttaagagtt catatcgaca attagggttt 60
acgacctcga tgttggatca ggacatccca atggtgcaga agctattaat ggttcgtttg 120
ttcaacgatt aaagtcctac gtgatctgag ttcagaccgg agcaatccag gtcggtttct 180
atctatttac aatttctccc agtacgaaag gacaagagaa atggagcctc cttaccataa 240
gtgctcccaa ccaatttatg aaaaaaaaaa aaaaaa 276

<210> 53
<211> 420
<212> DNA
<213> Rattus norvegicus

<400> 53
ggatggccct gtaagctagt cacttctagg actgtggcag gctttgatgg cccaatgcag 60
ttctctggag aaaactacct ttccccaagg catctgcacc cattgacaat ggtaatgtgc 120
ccatctctcc ttggtcctgc cctcaaccga tgcttttcca gtcagggttt tgtttttttgt 180
tttgttttgt gtacctcaac tgagttatga agatttgtac tggttttaca gatcatctca 240
tcgtatggat tagaacaagc ttcgtggtca gtttgctggg tgaccggcag acaccacaat 300
caaactagtc tgggaaaaac ctgctttttt gttgtaggtg ccacgtaacc ctgtcagttt 360
aacaaggaat gaccgtgcca ataaaccaat tctccctctg cttgaaaaaa aaaaaaaaaa 420

<210> 54
<211> 205
<212> DNA
<213> Rattus norvegicus

<400> 54
accccaacag actagagact ctaccgttcg tagtttatag catctctctc cggtattggc 60
aattcaccca cacaccttca gatgtgtctt cgggcgctct accattcaac ttgggaacta 120
cgagtaactc ccacacagcc tcatattata atcgggaaca aaaaaaccaa tctgtcaata 180
ttattgctaa aaaaaaaaaa aaaaa 205

<210> 55
<211> 142
<212> DNA
<213> Rattus norvegicus

<400> 55
aacagccaac catctaaaca tgaaaacaac gatgtcaaac taaataagga ctatatactt 60
gataatgttt tgctactctt gtcagacaat ggctataaac tgaattaggc agtcttaaca 120
aaaaataaaa aaaaaaaaaa aa 142

<210> 56
<211> 150
<212> DNA
<213> Rattus norvegicus

<400> 56
gacgtggnaa cagccngggg tggtggtggc cacgtctggt agcatgtgct gacctcacct 60
cttagccacg gtgtgacctt aagacaagtc atttagcttc caagtttcag ttccttgcag 120
cacctagatg acatttaaaa aaaaaaaaaa 150

<210> 57
<211> 298
<212> DNA
<213> Rattus norvegicus

<400> 57
gatagacgaa gcacactgcc tactcctatg cttgttcact ccatcgaaaa gtgatgaaac 60
ctgatggaga atgaacatat gcataccca gacattggag gccaggggaa cacataccaa 120
ggcatccagg atatgattcg tcacctgcgc atgattaatg gatgggctgt ggaggtgtaa 180
ctatgcctgc agttagctca gtctgactga acgactcgct tctcatatta gcacaccagc 240
tagcttaggg gacaggctcc agaataaagc cacttgtgtt ccaaaaaaaa aaaaaaaa 298

<210> 58
<211> 338
<212> DNA
<213> Rattus norvegicus

<400> 58
gactagtcag gtctcaacat ctacntgaca tggctggcac tgtgtgtatg tggtgcacaa 60
acacacgaag gcagaacacc taaaaggggt atatgtgtta tcatttaagt gtctctttaa 120
atgaaaagcc ttcaaccagg atttccctcc ttacaatata gatttgatgt ccaccctgtg 180
tcatgggaac tgagaggaag ggcagtataa atctgagagg ttcctttgtg tggtggaccc 240
cgaagaagaa agccccatgg ctgaacagct gttgtctcct cctaccccac agctttccct 300
aataaaggga ttgttatttt gaaaaaaaaa aaaaaaaa 338

<210> 59
<211> 108
<212> DNA
<213> Rattus norvegicus

<400> 59
ggaggcgncg tnantttcta tagtgcacta aacatctgct taaaagttct ttaattgggt 60
accatttctt caaataaaga atttgggtac ccaaaaaaaa aaaaaaaa 108

<210> 60
<211> 300
<212> DNA
<213> Rattus norvegicus

<400> 60
gaatatccag cacaaacaac tacngntttt accnnggggg acctgttcct cacatcagct 60
tagatatcta gtaagatccg tagntatata ctatgtgata cagcagttac acttaaggga 120
acnntgaaag ccgagcatgg caggtgnact ctcgattcgg tgatcaatgg tttcggtagn 180
gacatacatg tcgagngccg cgatgacagt tatatatgct cantcgcgtg gcatcgangt 240
attatagcaa tataggatac gataaanatg tcgccngcaa caccaaaaaa aaaaaacaaa 300

<210> 61
<211> 125
<212> DNA
<213> Rattus norvegicus

<400> 61
ctaaccagcn atatacgagg cngaggccgg cggatctctg agttagaagg ccagcctggt 60
ctacaacagt gagtnttcag gactgccagg ctatctacaa aacaaaaaac caaaaaaaaa 120
aacna 125

<210> 62
<211> 204
<212> DNA
<213> Rattus norvegicus

```
<400> 62
taccagacag ttgaactata gccttatgaa tttagacacg ctgctngttg taggtgtgta 60
caggccacct cccaaggcga ataactgaaa ctcgactgnc tggattgaag aaatgtgtgt 120
gttgctactg ttgcacatgt gtgcttatga cttgttgcag agggatttcc tattaaaaga 180
ctaatctgtc aaaaaaaaaa aaaa                                         204

<210> 63
<211> 111
<212> DNA
<213> Rattus norvegicus

<400> 63
gacgggagca ncagggnggg ttnnattcta ttnacgnttt tctcccagta cgaaaggaca 60
agagaaatgg agcctcctta ccataagtgc tcccaaccaa aaaaagaaaa a          111

<210> 64
<211> 150
<212> DNA
<213> Rattus norvegicus

<400> 64
gagaatacgg gagggggtga caccactcct gtggttagaa ctgctcctgc agtatttact 60
ccagtttcta aggctacaga tatagcatgt acattaaacc ctttgccctg tgaaataaag 120
ttatcttaca cgaaaaaaaa aaaaaaaaag                                   150
```

## Description

The present invention relates to the use of differentially expressed polynucleotide sequences or polypeptides for the characterisation of pain status or progression, a method for characterising pain, a method for identifying therapeutic agents for pain and the use of such sequences for the development of a medicament.

The effective treatment of pain requires an understanding of its physiology. It is well known, however, that stimuli, which activate pain receptors in one tissue, may not activate pain receptors in another. For example, pricking or cutting which causes pain in skin tissue does not cause pain in the stomach or intestine. The causes of pain in skeletal muscle, joints, and arteries can also differ. Principles of Neurology, 6"ed., Adams, R. D., et al., eds. (McGraw-Hill: 1997), pp. 133-134. Consequently, methods useful for relieving one type of pain are often less effective, or even ineffective, when applied to the alleviation of others. In general, neuropathic pain is persistent and is characterized by burning, gnawing, aching, shooting, or lancinating sensations. It is frequently associated with hyperesthesia, hyperalgesia, allodynia, and hyperpathia, and in some cases by sensory deficit or autonomic dysfunction. Unfortunately, and unlike other types of pain, neuropathic-pain tends to respond poorly to analgesic medication. Principles of Neurology, 6"ed., Adams, R. D., et al., eds. (McGraw-Hill: 1997), p. 140.

Depending on the nerves involved, a particular instance of neuropathic pain can be classified as a central or peripheral neuropathy. Central neuropathies arise from spinal cord, brainstem, thalamic, and cerebral damage or disease, while peripheral neuropathies arise from damage or disease of peripheral nerves. Specific peripheral neuropathies include, but are not limited to: thoracic outlet obstruction syndromes; compression and entrapment neuropathies such as ulnar nerve palsy, carpal tunnel syndrome, peroneal nerve palsy, radial nerve palsy; and Guillain-Barré syndrome. The Merck Manual, 16th ed., 1518-1522 (1992).

Neuropathic, or neurogenic, pain arises from the direct stimulation of nervous tissue. Neuropathic pain encompasses a wide variety of disorders involving single and multiple nerves. These include, but are not limited to, trigeminal neuralgia and disorders due to herpes zoster, diabetes, and trauma (including causalgia); spinal arachnoiditis and spinal cord injuries; and the thalamic pain syndrome of Dejerine-Roussy. Principles of Neurology, 6" ed., Adams, R. D., et al., ed., (McGraw-Hill: 1997), p. 140.

Neuropathic pain is caused by a variety of factors including, but not limited to: trauma caused by injury or surgical operation; tumors; bony hyperostosis; casts; crutches; prolonged cramped postures; hemorrhage into a nerve; exposure to cold or radiation; collagen-vascular disorders; infectious diseases such as Lyme disease, HIV; and Herpes zoster; toxins such as emetine, hexobarbital, barbital, chlorobutanol, sulfonamides, nitrofurantoin, the vinca alkaloids, heavy metals, carbon monoxide, triorthocresylphosphate, orthodinitrophenol, and other solvents and industrial poisons; autoimmune reactions; nutritional deficiency, and vitamin B deficiency in particular; and metabolic disorders such as hypothyroidism, porphyria, sarcoidosis, amyloidosis, uremia and diabetes. The Merck Manual, 16th ed., 1518 (1992).

Because so many causes of neuropathic pain exist, and because it tends to respond poorly to analgesic medication, the discovery of drugs that safely and effectively aid in its relief has been difficult.

Whereas acute pain is generally symptomatic of tissue damage or inflammation, and lasts only as long as the underlying cause remains, chronic pain may persist indefinitely in the absence, or after recovery of tissue pathology. It may occur as the aftermath of injury (e.g. to peripheral nerves or to the spinal cord), or in association with other disease states, such as herpes zoster or diabetes, but often it arises with no obvious cause. Chronic pain is often severe, leading to major disability and a high suicide rate, and it is common, with a prevalence of approximately 1% at the severe level. In contrast to acute pain, chronic pain is often unresponsive to conventional analgesic drugs (opiates and NSAIDs), and presents a difficult therapeutic problem, since its cause can usually not be resolved. Various other classes of drug, (e.g. tricyclic antidepressants, some anticonvulsant drugs, such as gabapentin, may be effective, but the therapeutic response is very variable and often accompanied with severe side effects.

A common feature of many clinical syndromes where chronic pain develops is the existence of previous nerve damage, affecting peripheral nerves, the spinal cord or (as in stroke) the brain, and the concept of neuropathic pain (i.e. pain arising as a consequence of neuronal damage) has become accepted as the underlying cause of many different chronic pain conditions seen in the clinic. Several animal models of neuropathic pain have been developed, which mimic many aspects of the clinical condition. These include lesions of the sciatic nerve (constriction or partial section), constriction or section of spinal nerves, ischaemic lesions of the spinal cord, induction of diabetic neuropathy, etc. and such models have been subjected to detailed study of the anatomical, biochemical and physiological changes that accompany the development of the pain state.

In the last several years a number of experimental models for neuropathic pain have been developed (Bennett and Xie (1988), Pain 33:87-107; Seltzer et al. (1990), Pain 43:205-218; Kim and Chung (1992), Pain 50:355-363; DeLeo et al. (1994) Pain 56:9-16; Na et al. (1994), Neurosci. Lett. 177:50-52). The availability of these different models provides an opportunity to investigate mechanisms of neuropathic pain. Finding common features in different models should provide better insight into the mechanisms critical for neuropathic pain, and comparison of the models should help to understand pain development and progression.

Kim et al. compared three of the models on basis of neuropathic pain behaviours and the effects of surgical sympathectomy (Kim et al. (1997), Exp. Brain Res. 113:200-206). They found that the models of Bennett, Seltzer and Kim & Chung (reference see above) result in a very similar general pattern and time course of evoked pain behaviour, whereby the Bennett model showed biggest behavioural signs for ongoing pain. The same results have been shown for the effects of sympathectomy on the behavioural signs of evoked and ongoing neuropathic pain, respectively. Thus these three models can be used to discover basic common features involved in neuropathic pain.

Further animal models for pain are considered in the article of Walker et al. (1999), Molecular Medicine Today 5:319-321, comparing models for different types of pain, which are acute pain, chronic / inflammatory pain and chronic / neuropathic pain, on the basis of behavioural signs.

Object of the present invention is to identify and characterise development, conditions and progression of pain on the molecular basis.

This object is met by the use of polynucleotide sequences selected from the group of sequences SEQ ID NO: 1 to 64 or homologues or fragments thereof or the according polypeptides for the characterisation of a) the status which elicits pain and / or b) the progression of the pathology of pain, whereby the characterisation is carried out outside of a living body.

Polynucleotide sequences SEQ ID NO:1 to 64 are expressed sequence tags (ESTs) representing genes which are differentially expressed under pain, particularly under neuropathic pain, in the models of Bennett, Seltzer and Kim & Chung (Bennett and Xie (1988), Pain 33:87-107, Seltzer et al. (1990), Pain 43:205-218 and Kim & Chung (1992), Pain 50:355-363).

In the following the models are designated as follows:

The model of Bennett is based on the chronic constriction injury by the loose ligation of the sciatic nerve. Therefore this model is designated as "CCI model".

The model of Seltzer et al. is based on the tight ligation of the partial sciatic nerve and is therefore designated as "PSL model".

The model of Kim & Chung is based on the tight ligation of spinal nerves and is therefore designated as "SNL model".

These designations correspond to the designations used in the cited literature.

The three models are explained in more detail in the literature and in the examples below.

The term "polynucleotide sequence" or "nucleic acid sequence" designates in the present application any DNA or RNA sequence, independent of the length. Thus this term can describe short sequences like PCR primers or probes for hybridisation, as well as whole genes or cDNA of these genes.

The term "polypeptide" or "amino acid sequence" designates a chain of amino acids, independent from their length, however, in any case more than one amino acid.

As "homologues" of polynucleotide sequences such polynucleotide sequences are designated which encodes the same type of protein as one of the polynucleotide sequences described herein. Accordingly as "homologues" of a polypeptide the polypeptides are designated which have an amino acid sequence, wherein at least 70 %, preferably 80 %, more preferably 90 % of the amino acids are identical to one of the proteins of the present invention and wherein the replaced amino acids preferably are replaced by homologous amino acids. As "homologous" amino acids are designated which have similar features concerning hydrophobicity, charge, steric features etc. Most preferred are amino acid sequences, containing the species- or family-dependent differences of the amino acid sequence. Particularly as "homologues" sequences are designated those which correspond to one of the cited sequences in another species or individual. For example if in the present invention a rat model is used and the cited polynucleotide sequence encodes the rat protein, the according polynucleotide sequence and protein of a mouse in a mouse model is designated as "homologue". Further splice variants and members of gene families are designated as homologues.

"Fragments" of a polynucleotide sequence are all polynucleotide sequences, which have at least 10 identical base pairs compared to one of the polynucleotide sequences shown in the present application or by the genes represented by these polynucleotide sequences. The term "fragment" encloses therefore such fragments as primers for PCR, probes for hybridisation, DNA fragments included in DNA vectors like plasmids, cosmids, BACs or viral constructs, as well as shortened splice variants of the genes identified herein. As a fragment of a protein (polypeptide) amino acid sequences are designated which have at least three amino acids, preferably at least 10 amino acids. Therefore fragments serving as antigens or epitopes are enclosed in this designation.

In the present application the term "sequence" is used when either a polynucleotide sequence (= nucleic acid sequence) or a polypeptide (= amino acid sequence) or a protein is meant. That means when it is irrelevant which type of sequence is used the type is not designated particularly, but with the more common term "sequence".

The basis of the models and methods described in the present application is the examination and determination of the expression of genes, which are differentially expressed under pain or during development of pain. Therefore for the examination each sequence can be used which allows the determination of the expression rate of the considered gene. Such a sequence can be at least one of the polynucleotide sequences SEQ ID NO. 1 to 64 or homologues or fragments thereof, as well as the polypeptides encoded thereby, however, just as well polynucleotide sequences and the according polypeptides can be used which are (parts of) the genes represented by the polynucleotide sequences SEQ ID NO. 1 to 64.

According to the invention it has been found, that the genes represented by the polynucleotide sequences SEQ ID NO. 1 to 64 are differentially expressed correspondingly in one, two or all three different models for pain, which are the above described models "CCI", "PSL", SNL".

Therefore the present invention provides sequences, which represent genes, which are differentially expressed under pain. Such polynucleotide sequences and the according polypeptides allow the determination and examination of pain development, conditions and progression. These sequences have not yet been regarded in relation to pain. For these examinations animal models can be used. As such a model any animal can be used wherein the necessary preparations can be carried out, however mammalian models are preferred. Even more preferred are rodents and lagomorpha, particularly preferred are rats, mice and rabbits. The most preferred animal model of the present invention is a rat model.

The sequences of the present invention further can be used for diagnosing a neuropathic pain status of a human outside of the living body by determining the expression levels of at least one of the cited sequences in comparison to the non-disease status. During treatment period of a patient the expression of the presently shown sequences can also be used for assessing the efficacy of pain treatment outside of the body. In this case blood, cerebrospinal fluid (CSF) or tissue is removed from the patient and expression is determined in the samples.

For determination and comparison of the expression levels of at least one of the genes identified in the present invention any of the commonly known methods can be used, either on RNA/cDNA level or on protein level. For example PCR, hybridisation, micro array based methods, western blot or 2-D protein gel analysis are suitable methods. One preferred method is the digital expression pattern display method (DEPD method), explained in detail in WO99/42610. The method used for determination of expression levels is not restrictive, as long as expressed amounts can be quantified.

The sequences of the present invention further can be used to develop new animal models for pain. By examination of the expression levels of at least one of the sequences shown it might be determined in several animals a procedure which is useful for generating a suitable animal model for different interesting conditions.

In such a newly generated animal model as well as in one of the known models the efficiency of compounds can be tested. Further as models for testing the efficiency of compounds assay systems can be used. Such assay systems may be *in vivo, ex vivo* or *in vitro* assays. In any case the models are contacted with the compound(s) to be tested and samples are obtained from these models, wherein expression levels of the sequences are determined and compared to the non-treated model.

Dependent from the used model the samples can be derived from whole blood, cerebrospinal fluid (CSF) or whole tissue, from cell populations isolated from tissue or blood or from single cell populations (i.e. cell lines).

In one embodiment of the invention cellular assays can be used. Preferred cells for cellular assays are eukaryotic cells, more preferably mammalian cells. Most preferred are neuronal-like cells, like SHSY5Y (neuroblastoma cell line), glial cell lines like TPH-1 and BV-2, astrocytic cell lines like U373MG and A7, or COS cells (African green monkey, kidney cells); CHO cells (Chinese hamster ovary), HEK-293 cells (human embryonic kidney).

Whereas the comparison of the expression levels (disease / non-disease status) of at least one of the provided sequences might give information about the examined disease status, it is preferred to determine the expression levels of more than one of the sequences simultaneously. Thus several combinations of the sequences can be used at different time points. By combination of several sequences a specific expression pattern can be determined indicating and / or identifying the conditions of the disease. The more expression rates are determined simultaneously, the more specific the result of the examination might be. However, good results can also be obtained by combination of only a few sequences. Therefore for the present invention it is preferred to compare the expression rates of at least two of the sequences provided herein, more preferred of at least 4, further more preferred of at least 6 of the sequences.

Since the presently provided sequences represent genes, which are differentially expressed, the expression rates of the single genes can be increased or decreased independently from each other. "Independently" in this context means that the expression rate of each of the genes can but need not be influenced by each other. In any case expression levels different from the non-disease status might be a hint to the disease status, which is examined.

The disease status, which is considered in the present invention is pain. The preferred types of pain are persistent / central pain, inflammatory pain (acute or chronic) or chronic pain. The most preferred type is neuropathic pain. Examples of such diseases are diabetic neuropathy, post-herpetic neuralgia, trigeminal neuralgia, cancer associated pain, spinal cord injury, multiple sclerosis, phantom pain, post-stroke pain, HIV associated pain, low back pain associated neuropathic pain, complex regional pain syndromes, like reflex sympathetic dystrophy and causalgia, myofacial syndromes or idiopathic pain conditions.

Independent of whether a pain status is diagnosed or characterised, a model for pain is characterised, the efficacy of pain treatment or the efficiency of a compound in a model shall be examined, the determination of the expression levels of at least one of the sequences is carried out outside of a living body. A method to obtain such results comprises

a) providing a sample comprising cells or body fluids expressing or containing one or more genes or gene products represented by polynucleotide sequences selected from the group of SEQ ID NO. 1 to 64 or homologues or fragments thereof
b) detecting expression of one or more of the genes in said cells;
c) comparing the expression of the genes in the test cells to the expression of the same genes in reference cells whose expression stage is known, and
d) identifying a difference in expression levels of the considered sequences, if present, in the test cell population and the reference cell population.

As mentioned above, detection of the expression of the genes can be carried out by any method known in the art. The method of detection is not limiting the invention.
Expression levels can be detected either on basis of the polynucleotide sequences or by detecting the according polypeptide, encoded by said polynucleotide sequence.
Preferred methods for detection and determination of the gene expression levels are PCR of cDNA, generated by reverse transcription of expressed mRNA, hybridisation of polynucleotides (Northern, Southern Blot systems, *In situ* hybridisation), DNA-micro-array based technologies, detection of the according peptides or proteins via e.g. Western Blot systems, 2-dimensional gel analysis, protein micro-array based technologies or quantitative assays like e.g. ELISA tests.
The most preferred method for quantitative analysis of the expression levels is the differential expression pattern display method (DEPD), described in detail in WO99/42610.
The sequences of the present invention can further be used for identifying therapeutic agents and their efficiency for treating pain. For example a method can be used comprising:

a) providing a test cell population comprising cells capable of expressing one or more genes represented by nucleic acid sequences selected from the group consisting of SEQ ID NO: 1 to 64 or homologues or fragments thereof
b) contacting said test cell population with the test therapeutic agent;
c) detecting the expression of one or more of the genes in said test cell population,
d) comparing the expression of the gene(s) in the test cell population to the expression of the gene(s) in a reference cell population whose disease stage is known; and
e) identifying a difference in expression levels of the considered sequences, if present, in the test cell population and the reference cell population, thereby identifying a therapeutic agent for treating pain.

Test cells can be obtained from a subject, an animal model or cell cultures of fresh cells or cell lines. Further *in vitro* assays may be used.
A method examining the different expression patterns of the differentially expressed genes therefore can be used for testing agents and compounds for their efficiency for treatment of pain. Which model is used is not relevant, as long as the model allows the determination of differences in expression amounts.
In such a model cells are contacted with the interesting agent or compound and expression of at least one of the genes considered in the present invention is determined in comparison to the expression of the same gene in cells which never have been contacted to the according agent / compound. Contacting the cells either can be effected by administering the agent / compound to an animal or by contacting isolated cells of tissue or blood or cells of cell lines in culture with the agent / compound.
By examination of the influence the considered agent / compound have to the expression of at least one of the genes the efficacy of the agent / compound can be estimated. This allows the decision whether it is worthwhile to develop a medicament containing such an agent or compound.
Whether the expression is determined on basis of mRNA generation or on basis of protein generation is not relevant, as long as the difference of the expression rate can be determined. Therefore the polynucleotide sequences, as well as the polypeptides or proteins shown in the present application can be used for the development of a medicament.
The development of a medicament can be desirable for example if the considered compound has an influence on the regulation of the expression rate or on the activity of any polynucleotide sequence or polypeptide of the present invention.
Said influence of a compound or agent can be examined by a method comprising contacting a sample comprising one of the nucleic acid sequences or of the polypeptides of the present invention with a compound that binds to said sequence in an amount sufficient to determine whether said compound modulates the activity of the polynucleotide or polypeptide sequence.

By such a method a compound or agent modulating the activity of any of the nucleic acid sequences or any polypeptides of the present invention can be determined.

Furthermore the sequences itself can be used as a medicament.

An example for such a use is the use of a polynucleotide sequence as an antisense agent. Antisense agents can hybridise to DNA or mRNA, inhibiting or decreasing transcription or translation, respectively. Thus, polynucleotide sequences of a gene, which is increased in expression rate under pain, can be used as antisense agents to decrease the expression rates of said gene. Further such polynucleotide sequences can be used for gene therapy.

Another example for such a use is the use of a polypeptide or a protein as a medicament. In case that the expression of a gene is decreased under pain and therefore not "enough" protein is provided by the body to maintain natural (healthy) conditions, said protein can be administered as a medicament.

A pharmaceutical composition comprising a polynucleotide sequence or a polypeptide according to the present invention can be any composition, which can serve as a pharmaceutical one. Salts or aids for stabilising the sequences in the composition preferably are present.

For the determination of the expression of the relevant genes the generated sequences have to be detected. Therefore several reagents can be used, which are for example specific radioactive or non-radioactive (e.g. biotinylated or fluorescent) probes to detect nucleic acid sequences by hybridisation, primer sets for the detection of one or several of the nucleic acid sequences by PCR, DNA microarrays, antibodies against one of the polypeptides, or epitopes or antibody or protein microarrays. Such reagents can be combined in a kit, which can be sold for carrying out any of the described methods.

Further the sequences defined in the present invention can be used to "design" new transgenic animals as models for pain. Therefore the animals are "created" by manipulating the genes considered in the present application in a way that their expression in the transgenic animal differs from the expression of the same gene in the wild type. In which "direction" the gene expression has to be manipulated (up- or downregulation) depends on the gene expression shown in the present application. Methods of gene manipulation and methods for the preparation of transgenic animals are commonly known to those skilled in the art.

For further examinations or experiments it might be desirable to include any of the nucleic acids of the present invention into a vector or a host cell. By including the sequences in a host cell for example cellular assays can be developed, wherein the genes, polynucleotide sequences and the according proteins / polypeptides further can be used or examined. Such vectors, host cells and cellular assays therefore shall be considered as to fall under the scope of the present invention.

Figures:

Figures 1 to 6 show comparison experiments of three pain models CCI, PSL and SNL. Expression levels of 6 differentially expressed sequences are represented. Each sequence is compared over a time period of 28 days to sham operated controls. Genes are described as differentially expressed when the sequence is up or down regulated at one time point in at least two of the three models. Over time the expression pattern can be determined as up-regulated in one to four time points; as down regulated in one to four time points or as mixed regulated if the type of regulation changes between up and down regulation at different time points.
X-axis describes the four time points analysed by DEPD, 1= day one post operation, 2= day 7 post operation, 3= day 14 post operation, 4=day 28 post operation. The Y-axis shows $\Delta$ h which represents the normalised difference of expression (peak height) of a certain transcript between a control group and a treated group. x-fold difference in gene expression is calculated by

$$\frac{1+\Delta h}{1-\Delta h}$$

0= no change to control, + = up regulation, - = down regulation; (0,2 = 1,5 fold; 0,3 = 1,86 fold; 0,4 = 2,33 fold; 0,5 = 3 fold).

The following examples are provided for illustration and are not intended to limit the invention to the specific example provided.

Examples:

Example 1: Preparation of rat models

A) The CCI model: (Bennett and Xie, Pain 1988, 33:87-107)

Nerve injuriy is created by loosely constrictive ligatures around the rat sciatic nerve (4 ligatures). The ligatures evoke intraneural edema, the swelling is opposed by the ligatures of the nerve strangulates. The constrictions remain for at least a month (hence "chronic constriction injury" CCI). It is known that the constriction injures nearly all of the nerves large myelinated axons; however, a variable but large percentage of the nerves unmyelinated axons remained intact. Although the nerve distal to the constriction is full of degeneration, the nerve proximal to the constriction appears normal, and there is no evidence of any primary afferent neuron dying. This model has a periphery that is innervated only by C-fibers and a greatly reduced number of A-delta fibers, and a spinal cord that is innervated by injured (but alive) A-beta low-threshold mechanoreceptors (AßLTMs), A-delta fibers (mostly injured, a few intact) and both injured and intact C-fiber afferents, many of which are nociceptors. This animal model provokes allodynia and hyperalgesia as well as spontaneous pain. Evidence of abnormal pain sensation is detected in the majority of CCI cases on the second PO day, and in nearly all cases by 5-7 days post injury. Abnormal pain sensation appears to reach peak severity in 10-14 days and to disappear in about 2 month when the pain is replaced by an apparently permanent state of hyperaesthesia.

In detail, male Lewis rats (150-350g body weight) were anaesthetised with sodium pentobartital (50mg/kg i. p.). Thereafter the common sciatic nerve on the left side was exposed at the level of the middle of the thigh by blunt dissection through the biceps femoris. Proximal to the sciatics trifurcation, about 7 mm of nerve was freed from adhering tissues and 4 ligatures (4.0 chromic gut) were tied loosely around it with 1 mm spacing. The length of nerve affected was 4-5 mm long. The desired degree of constriction retarded, but did not arrest, circulation through the superficial epineurial vasculature and sometimes produced a small, brief twitch in the muscle surrounding exposure. Control group animals were sham ligation (only left site), which represents the same operative procedure as the ligated one but without nerve ligation.

Four time points for gene expression profiling were chosen: 1day, 7 days, 14 days, and 28 days post operation. Animals were tested for mechanical allodynia (von Frey test) one day before surgery and within 30-60 min before tissue preparation (dorsal root ganglia, spinal cord and thalamus). Tissues were frozen on liquid nitrogen prior to RNA preparation.

B) The PSL model: Seltzer, Dubner, and Shir (Pain 1990, 43: 205-218)

Disorders in nocifensive behaviour following noxious and non-noxious stimuli begin hours after partial sciatic nerve injury and last for at 7 month. This model is characterized by complex combination of rapid onset, allodynia to touch, hyperalgesia, mirror image phenomena, and dependence on the sympathetic outflow. This model resembles therefore many of the symptoms described for causalgia in man. The rapid initiation of these disorders and their contralateral appearance suggest central reorganization. This model may serve as a model for sympathetically maintained pain (SMP)

In detail Lewis rats (male 150-350g body weight) were anaesthetised with sodium pentobartital (50mg/kg i. p.) followed by exposure of the left sciatic nerve at high-thigh level. Under 25x magnification, the dorsum of the nerve was carefully freed from surrounding tissues at a site near the trochanter just distal to the point at which the posterior biceps semitendinous ('PBST') nerve branches off the common sciatic nerve. An 8-0 silicon-treated silk suture was inserted into the nerve with a 3/8 curved, reversed-cutting mini-needle, and tightly ligated so that the dorsal 1/3 to ½ of the nerve thickness was trapped in the ligature.

Control group animals were sham ligated, which represents the same operative procedure as the ligated one but without nerve ligation.

Four time points for gene expression profiling were chosen: 1day, 7 days, 14 days, and 28 days post operation. Animals were tested for mechanical allodynia (von Frey test) one day before surgery and 30-60 min before tissue preparation (dorsal root ganglia, spinal cord and thalamus). Tissues were frozen on liquid nitrogen prior to RNA preparation.

C) The SNL model (Kim and Chung Pain 50 (1992) 355-363)

In this model a tight ligation of the left L5 and L6 spinal nerves or L5 spinal nerve alone is leading to a long-lasting hyperalgesia to noxious heat, at least 5 weeks of the affected foot. Long-lasting mechanical allodynia of the affected foot can be observed for at least 10 weeks. This model involves a complete ligation of spinal nerves L5 and L6 or only L5 (or L4), which is more reliable to other models where the number and types of ligated nerves are difficult to control

In detail, male Lewis rats (150-350g body weight) were anaesthetised with sodium pentobartital (50mg/kg i.p.). Thereafter the sciatic spinal nerve ligation of the spinal nerve L5 was performed on the left site of the animals. Control group animals were sham ligation (only left site), which represents the same operative procedure as the ligated one but without nerve ligation.
Four time points for gene expression profiling were chosen: 1day, 7 days, 14 days, and 28 days post operation. Animals were tested for mechanical allodynia (von Frey test) one day before surgery and 30-60 min before tissue preparation (dorsal root ganglia, spinal cord and thalamus). Tissues were frozen on liquid nitrogen prior to RNA preparation.

Example 2: Determination of expression levels

Gene expression profiling by DEPD-analysis starts with the isolation of 5-10µg total RNA. In a second step, double-stranded cDNA is synthesized. Through an enzymatic digest of the cDNA with three different type IIS restriction enzymes, three pools with short DNA-fragments containing single-stranded overhangs are generated. Afterwards, specific DNA-adaptor-molecules are ligated and in two subsequent steps 3.072 PCR reactions are performed by using 1024 different unlabelled 5'primer and a common FAM- fluorescent- labelled 3'-primer in the last PCR step. Subsequently, the 3072 PCR pools are analysed on an automatic capillary electrophoresis sequencer.
Differential gene expression pattern of single fragments are determined by comparison of normalized chromatogram peaks from the control groups and corresponding operated animals.

Example 3: Sequencing and Databank analysis of the obtained sequences

Differentially expressed peaks are confirmed on polyacrylamid gels by using radioactive labelled 3' primer instead of the FAM fluorescent primer. Differentially expressed bands are cut from the gel. After a short elution step in 60µl 10mM Tris pH8, fragments are re-amplified by PCR using the same primer as used in the DEPD analysis. Resulting PCR products are treated with a mixture of ExonucleaseI and shrimp alkaline phosphatase prior to direct sequencing. Sequencing reactions are performed by using DYEnamic-ET-dye terminator sequencing kit (Amersham) and subsequently analysed by capillary electrophoresis (Megabace 1000, Amersham).
Prior to a BLAST sequence analysis (Altschul et al. 1997, Nucleic Acids Res. 25:3389-3402) against Genbank ($1^{st}$ annotation, Table 1) and Unigene ($2^{nd}$ annotation, Table 1), all sequences are quality verified and redundant sequences or repetitive motifs are masked.
Results are shown in Table 1.

| Seq-ID | ascession number | name name | fragment length [bp] |
|---|---|---|---|
| 1 | | novel | 250 |
| 2 | Y00054 | HSC73 | 244 |
| 3 | BE112971 | UI-R-BJ1-awa-h-11-0-UI.s1 UI-R-BJ1 Rattus norvegicus cDNA clone UI-R-BJ1-awa-h-11-0-UI 3', mRNA sequence | 155 |
| 4 | BE928419 | RC0-CT0499-290800-024-c01<br>CT0499 Homo sapiens cDNA, mRNA sequence | 132 |
| 5 | AB030215 | COXII or Elf-1 (might be 3' similarity)<br>Rattus norvegicus mRNA for transcription factor Elf-1, complete cds. 1/2001 | 85 |
| 6 | BF288590 | EST453181 Rat Gene Index, normalized rat, Rattus norvegicus cDNA Rattus norvegicus cDNA clone RGIGV25, mRNA sequence. | 100 |
| 7 | BF288184 | EST452775 Rat Gene Index, normalized rat, Rattus norvegicus cDNA Rattus norvegicus cDNA clone RGIGQ58 3' sequence | 178 |
| 8 | | novel | 85 |
| 9 | AW534512 | UI-R-BS0-ans-d-12-0-UI.s1 UI-R-BS0 Rattus norvegicus cDNA clone UI-R-BS0-ans-d-12-0-UI 3', mRNA sequence. | 167 |
| 10 | M55424 | neurofilament NF-L | 150 |

(continued)

| Seq-ID | ascession number | name name | fragment length [bp] |
|---|---|---|---|
| 11 | AB023781 | cathespsin Y | 255 |
| 12 | X61479 | CSF-1 receptor | 158 |
| 13 | AJ001633 | Mus musculus mRNA for annexin III | 116 |
| 14 | M88347 | Rat pseudo-cystathionine beta-synthase mRNA, complete cds (type 4 of four alternatively spliced mRNAs). 4/1993 | 325 |
| 15 | AI058239 | EST49 Rat cochlea outer hair cells Lambda Zap Express Library Rattus norvegicus cDNA clone 300c 5', mRNA sequence. 3/1999 | 97 |
| 16 | BF286224 | EST450815 Rat Gene Index, normalized rat, Rattus norvegicus cDNA Rattus norvegicus cDNA clone RGIFN38 5' sequence | 76 |
| 17 | BF807839 | RC3-CI0042-111100-011-f06 CI0042 Homo sapiens cDNA, mRNA sequence | 167 |
| 18 | AW531794 | UI-R-C4-akz-b-06-0-UI.s1 UI-R-C4 Rattus norvegicus cDNA clone UI-R-C4-akz-b-06-0-UI 3', mRNA sequence. | 181 |
| 19 | | novel | 153 |
| 20 | X83231 | R.norvegicus mRNA for pre-alpha-inhibitor, heavy chain 3 | 97 |
| 21 | M72414 | MAP4 | 138 |
| 22 | V01227 | alpha tubulin | 209 |
| 23 | AK014144 | 1P: Mus musculus 13 days embryo head cDNA, RIKEN full-length enriched library, clone:3110038L02, full insert sequence. 7/2001 | 359 |
| 24 | AK004964 | Mus musculus adult male liver cDNA, RIKEN full-length enriched library, clone:1300011D16, full insert sequence. 7/2001 Weakly similar to T20253 hypothetical protein F53E4.1 -Caenorhabditis elegans [C.elegans] (unigene) | 167 |
| 25 | BE101201 | UI-R-BJ1-aua-c-03-0-UI.s1 UI-R-BJ1 Rattus norvegicus cDNA clone UI-R-BJ1-aua-c-03-0-UI 3', mRNA sequence. 6/2000 Weakly similar to T21321 hypothetical protein F25B3.3-Caenorhabditis elegans [C.elegans](unigene) | 121 |
| 26 | AF140232 | Rattus norvegicus calcium binding protein (S100A6), calcyclin | 246 |
| 27 | | novel | 113 |
| 28 | AY004290 | Rattus norvegicus scg10-like-protein | 254 |
| 29 | AI706278 | UI-R-AC0-yj-c-03-0-UI.s1 UI-R-AC0 Rattus norvegicus cDNA clone UI-R-AC0-yj-c-03-0-UI 3', mRNA sequence. 6/1999 ubiquitin carboxy-terminal hydrolase L1 (unigene) | 135 |
| 30 | X62322 | R.norvegicus mRNA for epithelin 1 and 2. 8/1992 | 462 |
| 31 | X16417 | Rat mRNA for beta-globin. 9/1993 | 208 |
| 32 | | novel | 211 |

(continued)

| Seq-ID | ascession number | name name | fragment length [bp] |
|---|---|---|---|
| 33 | AI044283 | UI-R-C1-kb-f-04-0-UI.s2 UI-R-C1 Rattus norvegicus cDNA clone UI-R-C1-kb-f-04-0-UI 3', mRNA sequence. 7/1999 Weakly similar to T46402 hypothetical protein DKFZp434H2121,1 [H.sapiens] (unigene) | 190 |
| 34 | S74324 | clone E501/543/588/5105, estrogen induced gene, rats, Sprague-Dawley, hypothalamus, mRNA Partial, 252 nt]. 4/1995 | 82 |
| 35 | BF410973 | UI-R-CN0-bmi-b-12-0-UI.s1 UI-R-CN0 Rattus norvegicus cDNA clone UI-R-CNO-bmi-b-12-0-UI 3', mRNA sequence. 11/2000 | 164 |
| 36 | | novel | 247 |
| 37 | M31038 | Rat MHC class I non-RT1.A alpha-1-chain mRNA, complete cds. 4/1993 | 233 |
| 38 | BF288184 | EST452775 Rat Gene Index, normalized rat, Rattus norvegicus cDNA Rattus norvegicus cDNA clone RGIGQ58 3' sequence, mRNA sequence. 11/2000 | 118 |
| 39 | AI058239 | EST49 Rat cochlea outer hair cells Lambda Zap Express Library Rattus norvegicus cDNA clone 300c 5', mRNA sequence. 3/1999 | 118 |
| 40 | D45249 | Rat mRNA for proteasome activator rPA28 subunit alpha, complete cds. 2/1999 | 299 |
| 41 | | novel | 112 |
| 42 | M17083 | Rat major alpha-globin mRNA, complete cds. 4/1993 | 336 |
| 43 | | novel | 191 |
| 44 | BF290323 | EST454914 Rat Gene Index, normalized rat, Rattus norvegicus cDNA Rattus norvegicus cDNA clone RGIHV04 3' sequence, mRNA sequence. 11/2000 | 129 |
| 45 | S56463 | HKII=hexokinase II [rats, epididymal fat pad, mRNA Partial, 266 nt, segment 1 of 2]. 6/1993 | 230 |
| 46 | | novel | 119 |
| 47 | | novel | 122 |
| 48 | L22643 | Rat anti-acetylcholine receptor antibody gene, kappa-chain, VJC region, complete cds. 7/1996 | 301 |
| 49 | AF028784 | Rattus norvegicus glial fibrillary acidic proteins alpha and delta (GFAP) gene, alternatively spliced products, complete cds. 6/1999 | 473 |
| 50 | | novel | 216 |
| 51 | BF557085<br>HSA292757 | UI-R-E1-go-a-09-0-UI.r1 UI-R-E1 Rattus norvegicus cDNA clone UI-R-EI-go-a-09-0-UI 5', mRNA sequence. 12/2000 Moderately similar to TBB1 RAT TUBULIN BETA CHAIN [R.norvegicus] (unigene) | 446 |
| 52 | AB010743 | Rattus norvegicus mRNA for UCP2, complete cds. 2/1999 | 305 |
| 53 | X82396 | R.norvegicus mRNA for cathepsin B. 9/1996 | 420 |

(continued)

| Seq-ID | ascession number | name name | fragment length [bp] |
|---|---|---|---|
| 54 | Z12298 | R.rattus mRNA for dermatan sulfate proteoglycan-II (decorin). 2/1997 | 205 |
| 55 | D90035 | Rattus norvegicus mRNA for calcineurin A alpha, complete cds. 7/1999 | 142 |
| 56 | D83349 | Rat mRNA for short type PB-cadherin, complete cds. 2/1999 | 150 |
| 57 | X74125 | R.norvegicus mRNA for NAD+-isocitrate dehydrogenase, gamma subunit. 7/1995 | 298 |
| 58 | BF407932 | UI-R-BJ0p-ait-h-01-0-UI.s2 UI-R-BJ0p Rattus norvegicus cDNA clone UI-R-BJ0p-ait-h-01-0-UI 3', mRNA sequence. 11/2000 | 338 338 |
| 59 | M18053 | Rat ferritin heavy subunit gene | 151 |
| 60 | | novel | 300 |
| 61 | | novel | 125 |
| 62 | Z22593 | M.musculus fibrillarin mRNA. | 204 |
| 63 | AI058239 | EST49 Rat cochlea outer hair cells Lambda Zap Express Library Rattus norvegicus cDNA clone 300c 5', mRNA sequence. 3/1999 | 111 |
| 64 | AF093567 | 1P: Rattus norvegicus myocilin mRNA, complete cds. 8/1999 (Myoc/tigr) | 150 |

Example 4: Comparison of differentially expressed sequences in three model systems.

Four time points for gene expression profiling were chosen for all three models: 1day, 7 days, 14 days, and 28 days post operation. Behavioural testing of the animals was performed one day before operation and 30-60 min prior to the tissue preparation. After DEPD analysis differentially expressed peaks obtained for the three different pain models were compared to identify overlapping gene expression patterns.

Results are shown in Table 2. For 6 selected sequences the regulation is further shown in Figures 1 to 6 ( Regulation of Seq No: 1, 18, 20, 22, 30 and 59)

| Seq-ID No. | Regulation |
|---|---|
| 1 | up |
| 2 | up |
| 3 | up |
| 4 | down |
| 5 | mixed |
| 6 | up |
| 7 | down |
| 8 | up |
| 9 | mixed |
| 10 | down |
| 11 | up |
| 12 | up |

(continued)

| Seq-ID No. | Regulation |
|---|---|
| 13 | up |
| 14 | up |
| 15 | down |
| 16 | mixed |
| 17 | up |
| 18 | down |
| 19 | down |
| 20 | up |
| 21 | up |
| 22 | mixed |
| 23 | down |
| 24 | down |
| 25 | up |
| 26 | mixed |
| 27 | mixed |
| 28 | mixed |
| 29 | mixed |
| 30 | up |
| 31 | mixed |
| 32 | mixed |
| 33 | up |
| 34 | up |
| 35 | up |
| 36 | up |
| 37 | up |
| 38 | down |
| 39 | mixed |
| 40 | up |
| 41 | down |
| 42 | up |
| 43 | mixed |
| 44 | mixed |
| 45 | up |
| 46 | down |
| 47 | up |
| 48 | up |
| 49 | up |
| 50 | down |

(continued)

| Seq-ID No. | Regulation |
|---|---|
| 51 | mixed |
| 52 | up |
| 53 | up |
| 54 | mixed |
| 55 | up |
| 56 | mixed |
| 57 | mixed, |
| 58 | down |
| 59 | up |
| 60 | up |
| 61 | up |
| 62 | up |
| 63 | mixed |
| 64 | up |

For each DNA fragment, gene expression patterns obtained in the three pain models were matched. "Up", "down", and "mixed" regulation is defined as overlapping pattern in at least two of the three models at one or more time points.

## Claims

1. Use of polynucleotide sequences selected from the group of sequences SEQ ID NO:1 to 64 or homologues or fragments thereof or the according polypeptides for the characterisation of (a) the status which elicits pain and / or (b) the progression of the pathology of pain, whereby the characterisation is carried out outside of a living body.

2. Use of polynucleotide sequences according to claim 1, **characterised in that** the sequences are mammalian sequences.

3. Method for characterising a pain status, comprising the comparison of the expression of genes represented by at least one of the sequences selected of the group of SEQ ID NO.1 to 64 or homologues or fragments thereof to non-disease status.

4. Method according to claim 3, for diagnosing the pain status outside of the living body.

5. Method according to claim 3, for assessing the efficacy of pain treatment outside of a living body.

6. Method according to claim 3, for characterisation of animal models for pain.

7. Method according to claim 3, for determining the efficiency of compounds in a model for pain.

8. Method according to claim 3 to 7, whereby the expression is determined from in vivo samples, in vitro samples, or ex vivo samples.

9. Method according to claim 8, whereby the samples are derived from whole tissues, blood, cerebrospinal fluid (CSF), from cell populations isolated from tissues, blood or CSF or from cell lines.

10. Method according to claim 3 to 9, whereby at least a combination of 2 sequences are compared to non-disease status.

**11.** Method according to claim 3 to 9, whereby at least a combination of 4 sequences are compared to non-disease status.

**12.** Method according to claim 3 to 9, whereby at least a combination of 6 sequences are compared to non-disease status.

**13.** Method according to claim 3 to 12, whereby the considered gene expression is increased compared to non-disease status.

**14.** Method according to claim 3 to 12, whereby the considered gene expression is decreased compared to non-disease status.

**15.** Method according to claim 3 to 12, whereby the considered gene expression for at least one sequence is increased and for at least one sequence is decreased compared to non-disease status.

**16.** The method according to any of claims 3 to 15 comprising:

a) providing a sample comprising cells or body fluids expressing or containing one or more genes or gene products represented by polynucleotide sequences selected from the group of SEQ ID NO. 1 to 64 or homologues or fragments thereof
b) detecting expression of one or more of the genes in said cells;
c) comparing the expression of the genes in the test cells to the expression of the same genes in reference cells whose expression stage is known, and
d) identifying a difference in expression levels of the considered sequences, if present, in the test cell population and the reference cell population.

**17.** A method of identifying a test therapeutic agent for treating pain in a subject, the method comprising:

a) providing a test cell population comprising cells capable of expressing one or more genes represented by nucleic acid sequences selected from the group consisting of SEQ ID NO: 1 to 64 or homologues or fragments thereof
b) contacting said test cell population with the test therapeutic agent;
c) detecting the expression of one or more of the genes in said test cell population,
d) comparing the expression of the gene(s) in the test cell population to the expression of the gene(s) in a reference cell population whose disease stage is known; and
e) identifying a difference in expression levels of the considered sequences, if present, in the test cell population and the reference cell population, thereby identifying a therapeutic agent for treating pain.

**18.** Use or method according to any of claims 1 to 17, whereby the pain is neuropathic pain.

**19.** Method according to claim 3 to 18, whereby the gene expression is considered or determined by PCR of cDNA, hybridisation of a sample DNA or by detecting the according protein

**20.** Use of at least one of the polynucleotide sequences of genes represented by polynucleotide sequences selected from SEQ ID NO. 1 to 64 or homologues or fragments thereof or of a protein encoded by any gene represented by any of the polynucleotide sequences selected from SEQ ID NO. 1 to 64 or homologues or fragments thereof for the development of a medicament.

**21.** Isolated nucleic acid sequences comprising a nucleic acid sequence of a gene that is one of the genes represented by the polynucleotide sequences selected of SEQ ID NO. 1 to 64, or the complement of the nucleic acid sequence.

**22.** An isolated polypeptide comprising an amino acid sequence encoded by one of the genes represented by the nucleic acid sequences of SEQ ID NO. 1 to 64.

**23.** Use of an isolated nucleic acid sequence according to claim 21 or an isolated polypeptide according to claim 22 as a medicament.

**24.** A pharmaceutical composition comprising any of the nucleic acid sequences of claim 21 or any of the polypeptides of claim 23.

**25.** A kit comprising one or more reagents for detecting one or more genes represented by nucleic acid sequences selected from the group consisting of SEQ ID NO. 1 to 64 or homologues or fragments thereof.

**26.** A vector comprising any nucleic acid sequence of claim 21.

**27.** A host cell comprising any isolated nucleic acid sequence of claim 21 or a vector of claim 26.

**28.** An antibody that selectively binds to a polypeptide of claim 22, and fragments, homologues, analogues and derivatives of the antibody.

**29.** A transgenic animal wherein at least one of the sequences corresponding to the sequences represented by any of the SEQ ID NO: 1 to 64 is manipulated in the animal in comparison to the wild type.

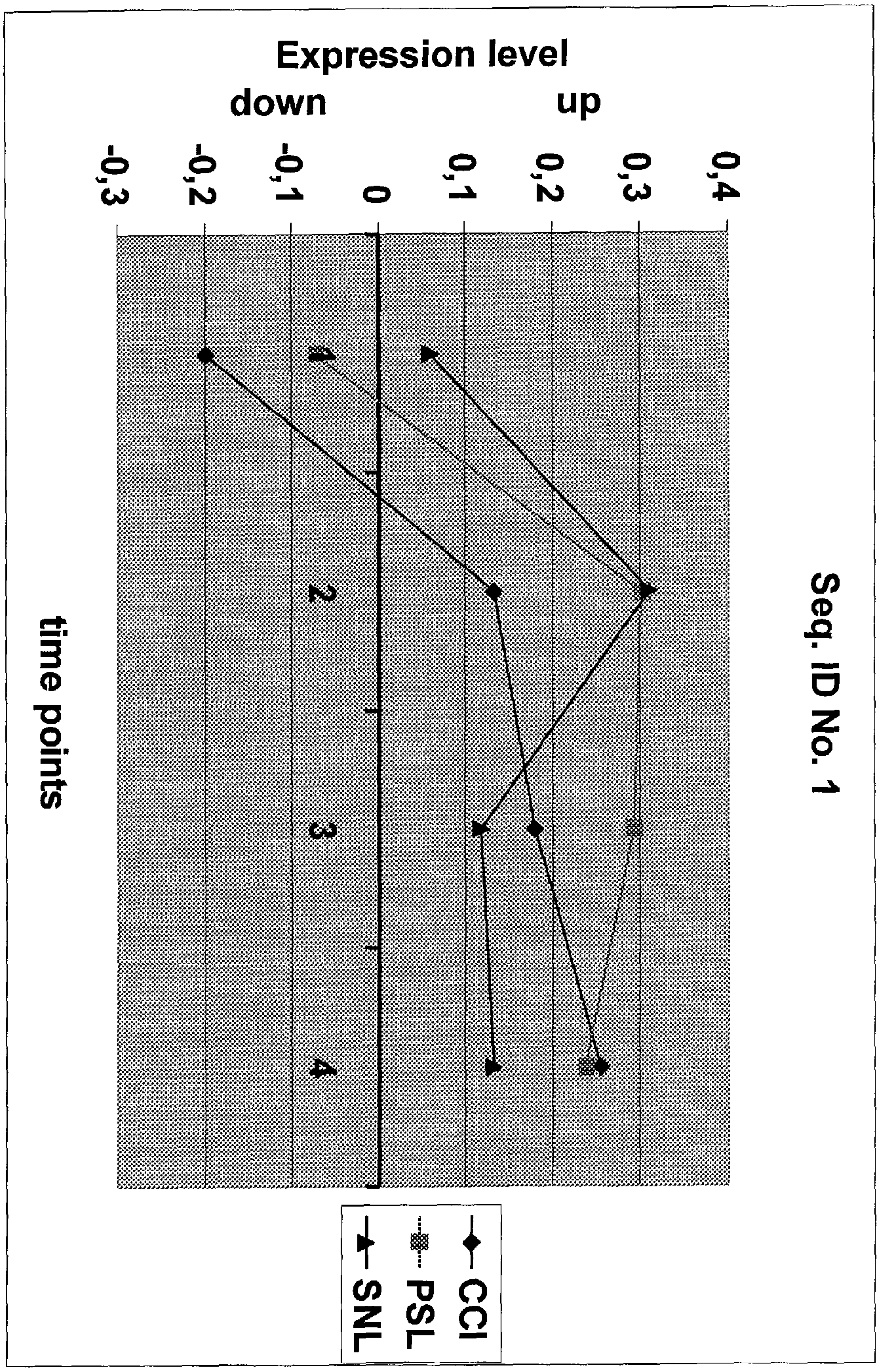
Seq. ID No. 1
Expression level
up
down
0,4
0,3
0,2
0,1
0
-0,1
-0,2
-0,3
1
2
3
4
time points
CCI
PSL
SNL

Figure 1

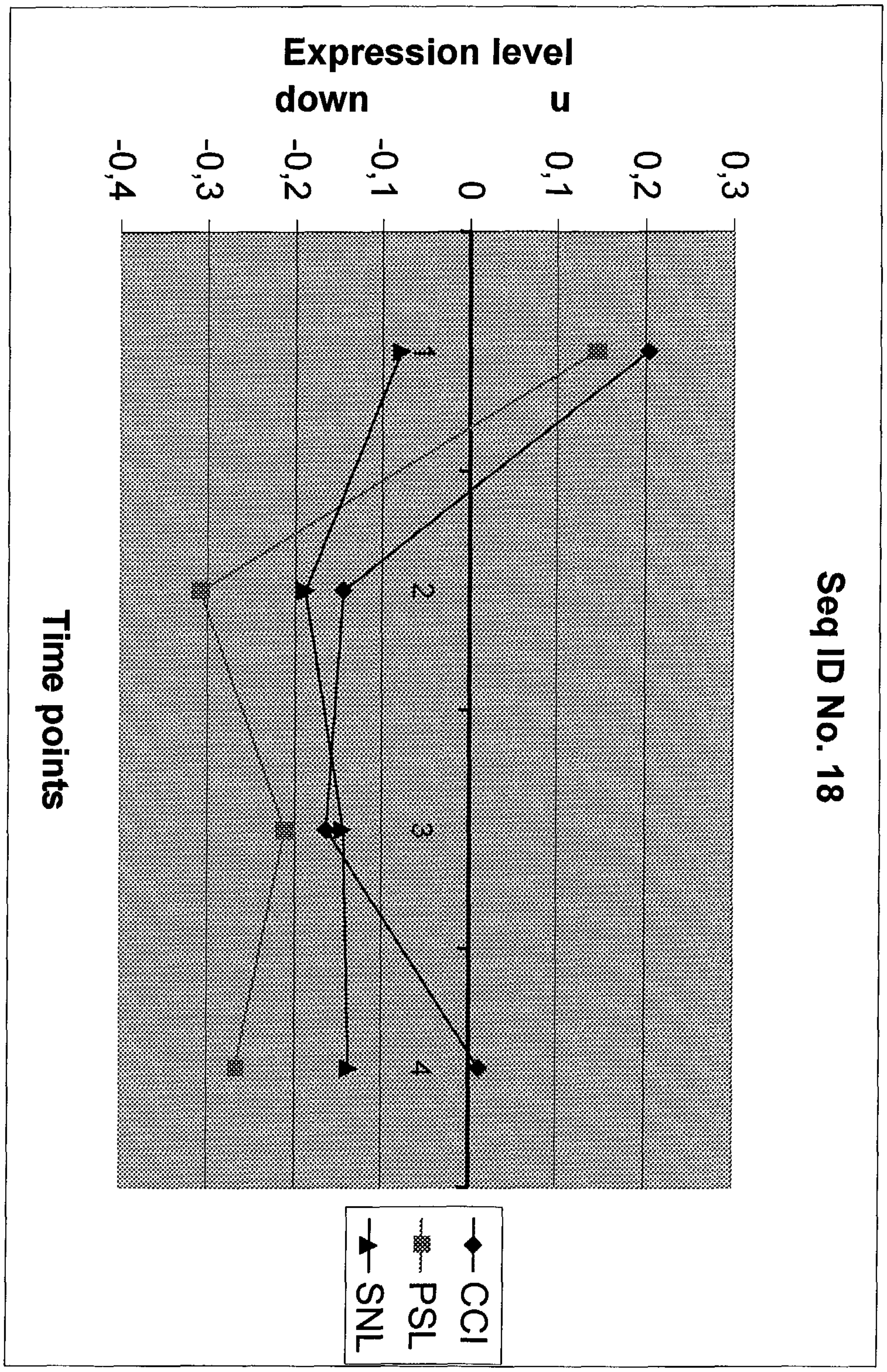
Seq ID No. 18
Expression level
down
u
0,3
0,2
0,1
0
-0,1
-0,2
-0,3
-0,4
1
2
3
4
Time points
CCI
PSL
SNL

Figure 2

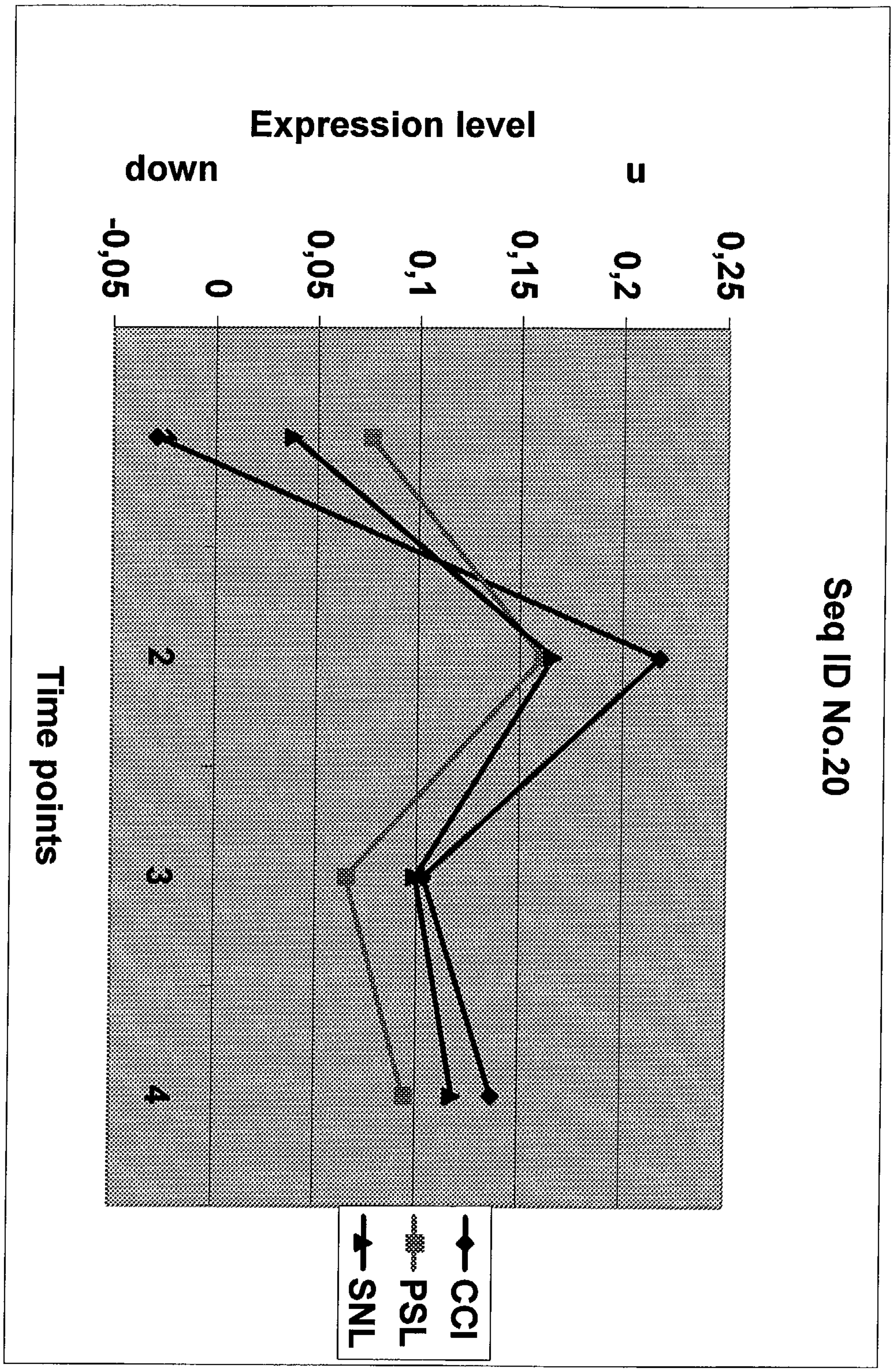
Seq ID No.20
Expression level
u
down
-0,05
0
0,05
0,1
0,15
0,2
0,25
1
2
3
4
Time points
CCI
PSL
SNL

Figure 3

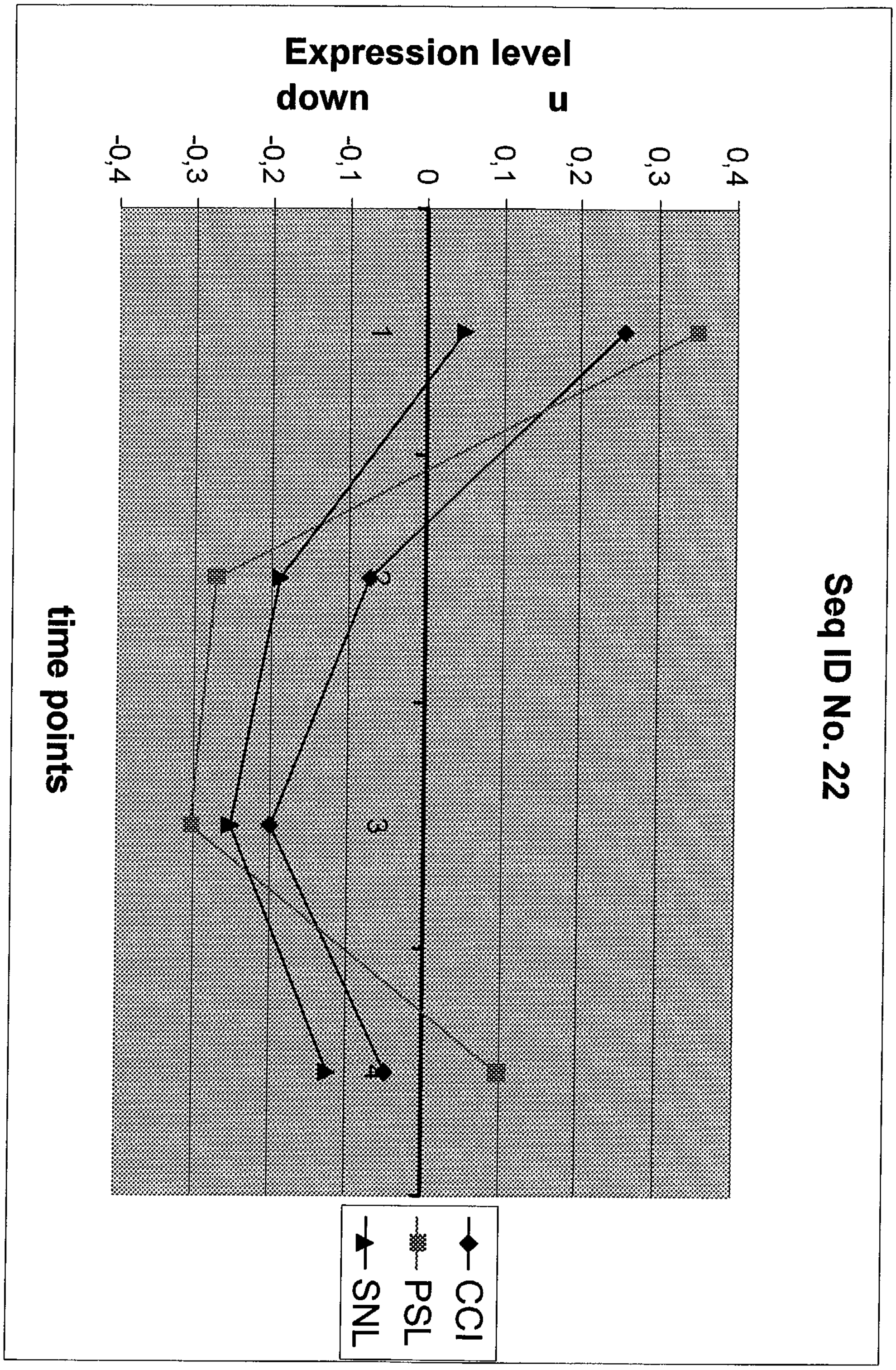
Seq ID No. 22
Expression level
down
u
0,4
0,3
0,2
0,1
0
-0,1
-0,2
-0,3
-0,4
1
2
3
4
time points
CCI
PSL
SNL

Figure 4

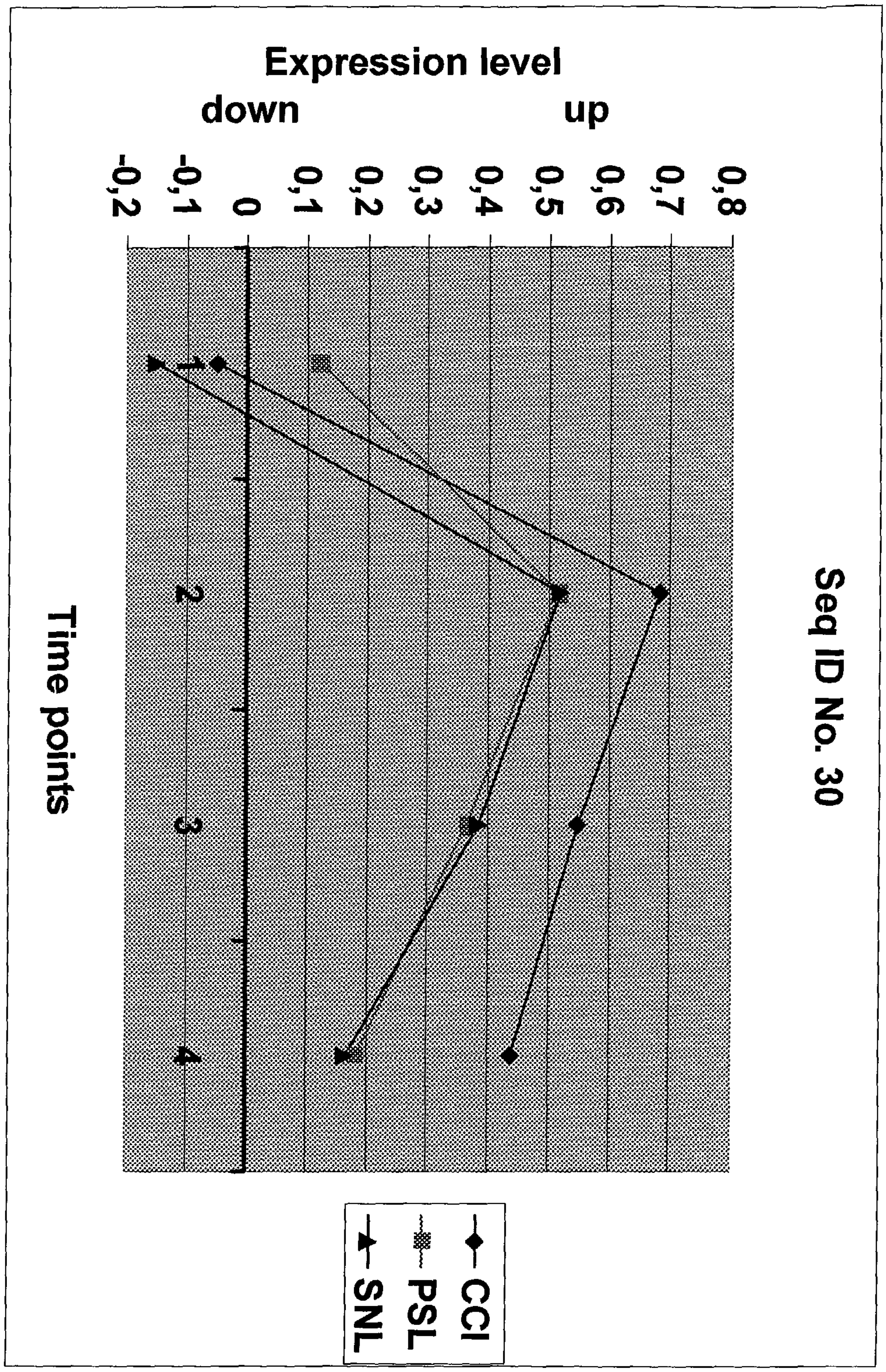
Seq ID No. 30
Expression level
up
down
0,8
0,7
0,6
0,5
0,4
0,3
0,2
0,1
0
-0,1
-0,2
1
2
3
4
Time points
CCI
PSL
SNL

Figure 5

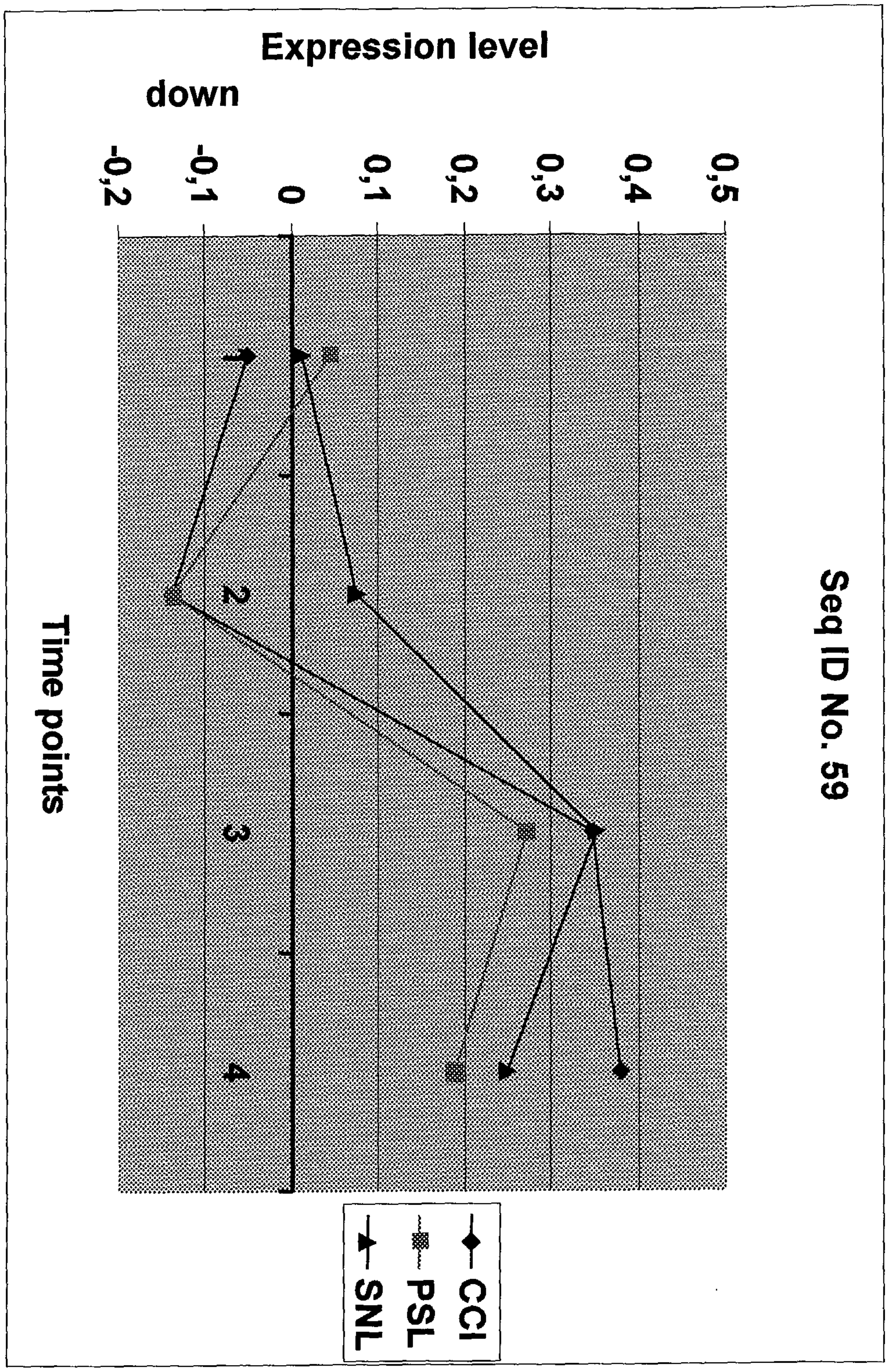
Seq ID No. 59
Expression level
down
0,5
0,4
0,3
0,2
0,1
0
-0,1
-0,2
1
2
3
4
Time points
CCI
PSL
SNL

Figure 6

European Patent Office

# EUROPEAN SEARCH REPORT

Application Number
EP 02 00 3401

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | NEWTON R A ET AL: "Identification of differentially expressed genes in dorsal root ganglia following partial sciatic nerve injury." NEUROSCIENCE, vol. 95, no. 4, 30 December 2000 (2000-12-30), pages 1111-1120, XP002196990 ISSN: 0306-4522 * the whole document * --- | 1-29 | C12Q1/68 |
| X | KIM DONG-SUN ET AL: "Differentially expressed genes in rat dorsal root ganglia following peripheral nerve injury." NEUROREPORT, vol. 12, no. 15, 2001, pages 3401-3405, XP001068889 ISSN: 0959-4965 * the whole document * --- | 1-29 | |
| X | ERLANDER M G ET AL: "DNA microassays and differential display identify numerous differentially-expressed mRNAs in DRG neurons of a neuropathic pain model." SOCIETY FOR NEUROSCIENCE ABSTRACTS, vol. 24, no. 1-2, 1998, page 1388 XP001079022 28th Annual Meeting of the Society for Neuroscience, Part 2;Los Angeles, California, USA; November 7-12, 1998 ISSN: 0190-5295 * the whole document * --- -/-- | 1-29 | TECHNICAL FIELDS SEARCHED (Int.Cl.7) C12Q |

~~The present search report has been drawn up for all claims~~

| Place of search | Date of completion of the search | Examiner |
|---|---|---|
| MUNICH | 22 May 2002 | Madlener, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 00 3401

## DOCUMENTS CONSIDERED TO BE RELEVANT

| Category | Citation of document with indication, where appropriate, of relevant passages | Relevant to claim | CLASSIFICATION OF THE APPLICATION (Int.Cl.7) |
|---|---|---|---|
| X | ROGERS K E ET AL: "PCR differential display identifies rat dorsal root ganglia mRNAs which are regulated in a neuropathic pain model." SOCIETY FOR NEUROSCIENCE ABSTRACTS, vol. 22, no. 1-3, 1996, page 965 XP001069782 26th Annual Meeting of the Society for Neuroscience;Washington, D.C., USA; November 16-21, 1996 ISSN: 0190-5295 * the whole document * --- | 1-29 | |
| X | GILLEN C ET AL: "DIFFERENTIALLY EXPRESSED GENES AFTER PERIPHERAL NERVE INJURY" JOURNAL OF NEUROSCIENCE RESEARCH, WILEY-LISS, US, vol. 42, no. 2, 1 October 1995 (1995-10-01), pages 159-171, XP000568934 ISSN: 0360-4012 * the whole document * --- | 1-29 | TECHNICAL FIELDS SEARCHED (Int.Cl.7) |
| X | LEON DE M ET AL: "IDENTIFICATION OF TRANSCRIPTIONALLY REGULATED GENES AFTER SCIATIC NERVE INJURY" JOURNAL OF NEUROSCIENCE RESEARCH, WILEY-LISS, US, vol. 29, no. 4, 1 August 1991 (1991-08-01), pages 437-448, XP000568933 ISSN: 0360-4012 * the whole document * --- -/-- | 1-29 | |

~~The present search report has been drawn up for all claims~~

| Place of search | Date of completion of the search | Examiner |
|---|---|---|
| MUNICH | 22 May 2002 | Madlener, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

# EUROPEAN SEARCH REPORT

Application Number
EP 02 00 3401

| DOCUMENTS CONSIDERED TO BE RELEVANT | | | |
|---|---|---|---|
| Category | Citation of document with indication, where appropriate, of relevant passages | Relevant to claim | CLASSIFICATION OF THE APPLICATION (Int.Cl.7) |
| X | MACDONALD RACHEL ET AL: "Determination of changes in mRNA expression in a rat model of neuropathic pain by TaqmanTM quantitative RT-PCR." MOLECULAR BRAIN RESEARCH, vol. 90, no. 1, 2001, pages 48-56, XP002196992 ISSN: 0169-328X * the whole document * | 1-29 | |
| A | GREEN CD ET AL.: "Open systems: panoramic views of gene expression" JOURNAL OF IMMUNOLOGICAL METHODS, vol. 250, 2001, pages 67-79, XP002196993 * page 76, right-hand column, last paragraph - page 77 * | 1-29 | |
| A | MOGIL JEFFREY S: "The genetic mediation of individual differences in sensitivity to pain and its inhibition." PROCEEDINGS OF THE NATIONAL ACADEMY OF SCIENCES OF THE UNITED STATES, vol. 96, no. 14, 6 July 1999 (1999-07-06), pages 7744-7751, XP002196994 July 6, 1999 ISSN: 0027-8424 * the whole document * | 1-29 | TECHNICAL FIELDS SEARCHED (Int.Cl.7) |
| A | BRIDGES D ET AL.: "Mechanisms of neuropathic pain" BR J ANAESTH, vol. 87, no. 1, July 2001 (2001-07), pages 12-26, XP001068882 * the whole document * | 1-29 | |

~~The present search report has been drawn up for all claims~~

| Place of search | Date of completion of the search | Examiner |
|---|---|---|
| MUNICH | 22 May 2002 | Madlener, M |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

European Patent Office

Application Number

EP 02 00 3401

## CLAIMS INCURRING FEES

The present European patent application comprised at the time of filing more than ten claims.

☐ Only part of the claims have been paid within the prescribed time limit. The present European search report has been drawn up for the first ten claims and for those claims for which claims fees have been paid, namely claim(s):

☐ No claims fees have been paid within the prescribed time limit. The present European search report has been drawn up for the first ten claims.

## LACK OF UNITY OF INVENTION

The Search Division considers that the present European patent application does not comply with the requirements of unity of invention and relates to several inventions or groups of inventions, namely:

see sheet B

☐ All further search fees have been paid within the fixed time limit. The present European search report has been drawn up for all claims.

☐ As all searchable claims could be searched without effort justifying an additional fee, the Search Division did not invite payment of any additional fee.

☐ Only part of the further search fees have been paid within the fixed time limit. The present European search report has been drawn up for those parts of the European patent application which relate to the inventions in respect of which search fees have been paid, namely claims:

☒ None of the further search fees have been paid within the fixed time limit. The present European search report has been drawn up for those parts of the European patent application which relate to the invention first mentioned in the claims, namely claims:

1-29 (partly)

European Patent Office

**LACK OF UNITY OF INVENTION**
**SHEET B**

Application Number
EP 02 00 3401

The Search Division considers that the present European patent application does not comply with the requirements of unity of invention and relates to several inventions or groups of inventions, namely:

1. Claims: Claims 1-29 (partly)

   Uses, methods, products and compositions according to claims 1-29 insofar as they relate to SEQ.ID.NO.1.

2. Claims: Claims 1-29 (partly)

   Uses, methods, products and compositions according to claims 1-29 insofar as they relate to SEQ.ID.NO.18.

3. Claims: Claims 1-29 (partly)

   Uses, methods, products and compositions according to claims 1-29 insofar as they relate to SEQ.ID.NO.20.

4. Claims: Claims 1-29 (partly)

   Uses, methods, products and compositions according to claims 1-29 insofar as they relate to SEQ.ID.NO.22.

5. Claims: Claims 1-29 (partly)

   Uses, methods, products and compositions according to claims 1-29 insofar as they relate to SEQ.ID.NO.30.

6. Claims: Claims 1-29 (partly)

   Uses, methods, products and compositions according to claims 1-29 insofar as they relate to SEQ.ID.NO.59.

7. Claims: Claims 1-29 (partly)

   Uses, methods, products and compositions according to claims 1-29 insofar as they relate to SEQ.ID.NO.2.

   ... Inventions 8-64: analogous subject-matter as above relating to SEQ.ID.NOs. 3-17, 19, 21, 23-29, 31-58 and 60-64.